(19) Europäisches Patentamt
European Patent Office
Office européen des brevets

(11) Publication number: 0 374 789 A1

(12) **EUROPEAN PATENT APPLICATION**

(21) Application number: 89123374.4

(22) Date of filing: 18.12.89

(51) Int. Cl.5: C07D 311/30, C07D 405/10, //A61K31/35

(30) Priority: 19.12.88 JP 320000/88

(43) Date of publication of application: 27.06.90 Bulletin 90/26

(84) Designated Contracting States: DE FR GB IT

(71) Applicant: KYOWA HAKKO KOGYO CO., LTD.
6-1, Ohtemachi Itchome
Chiyoda-ku Tokyo(JP)

(72) Inventor: Shida, Yasushi
410-1, Nameri Nagaizumi-cho
Sunto-gun Shizuoka-ken(JP)
Inventor: Sagaya, Toru
696-2-601, Mitsugarasu-cho
Nara-shi Naraken(JP)
Inventor: Gomi, Katsushige
1188, Shimotogari Nagaizumi-cho
Sunto-gun Shizuoka-ken(JP)
Inventor: Kasai, Masaji
3-12-15, Kugenuma Matsugaoka
Fujisawa-shi Kanagawa-ken(JP)
Inventor: Morimoto, Makoto
203-5, Shimotogari Nagaizumi-cho
Sunto-gun Shizuoka-ken(JP)

(74) Representative: Casalonga, Axel et al
BUREAU D.A. CASALONGA - JOSSE
Morassistrasse 8
D-8000 München 5(DE)

(54) **5- Aminoflavone derivatives.**

(57) Novel 5-aminoflavone derivatives represented by formula (I) and pharmaceutically acceptable salts thereof are disclosed. The 5-aminoflavone derivatives have anticellular activity and are expected to be useful as antitumor agents.

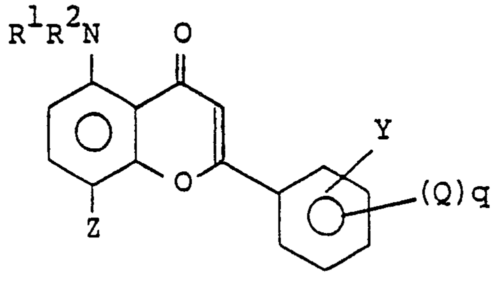

(I)

EP 0 374 789 A1

# 5-AMINOFLAVONE DERIVATIVES

## BACKGROUND OF THE INVENTION

The present invention relates to novel 5-aminoflavone derivatives having anticellular activity.

As derivatives of flavone (2-phenyl-4H-benzopyran-4-one) having an amino group at the 5-position, there have been disclosed compounds having hydroxyl at the 6-position [Chemical Abstracts, 41, 120f (1947)], compounds having alkoxy at the 3-position which exhibit antiviral activity (EP-A-233105) and compounds having a carboxylic acid residue or a group associated therewith on the phenyl ring which exhibit activities such as antiallergic activity (GB-A-1461777).

Further, as flavone derivatives showing anticellular activity, there are known flavone carboxylic acid derivatives (U.S. Patent No. 4,831,052), 5,7-dihydroxy-3′,4′,6,8-tetramethoxyflavone (hymenoxin) [Chem. Pharm. Bull., 36, 4849 (1988)], 3,5,3′-trihydroxy-6,7,4′-trimethoxyflavone (eupatin) and 3,3′-dihydroxy-5,6,7,4′-tetramethoxyflavone (eupatoretin) [J. Org. Chem., 34, 1460 (1969)], and flavone acetic acid derivatives [Eur. J. Med. Chem. - Chim. Ther., 20, 393 (1985)], 4′,5,7-trihydroxyflavone [Res. Commun. Chem. Pathol. Pharmacol., 64, 69 (1989)] and the like. However, none of these compounds possess an amino group at the 5-position.

## SUMMARY OF THE INVENTION

An object of the present invention is to provide novel 5-aminoflavone derivatives, based on the finding that 5-aminoflavone derivatives have anticellular activity.

The present invention relates to 5-aminoflavone derivatives represented by formula (I) and their pharma ceutically acceptable salts. [The derivatives are hereinafter referred to as Compounds (I); the same applies to the compounds of other formula numbers.]

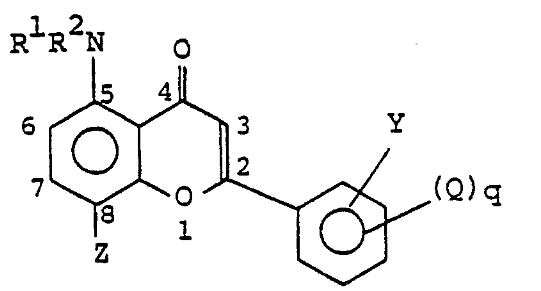

( I )

In the formula, $R^1$ and $R^2$ independently represent a member selected from the group consisting of hydrogen, lower alkyl, cycloalkyl, lower alkanoyl, amino, lower alkanoylamino, benzyl and $-(CH_2)_nX$ wherein X represents a member selected from the group consisting of hydroxyl, lower alkanoyloxy, lower alkoxyl, cycloalkyloxy, benzyloxy, mercapto, lower alkylthio, halogen, carboxyl, lower alkoxycarbonyl, lower alkylsulfonyloxy, trifluoromethanesulfonyloxy, optionally substituted arylsulfonyloxy, $-CONR^3R^4$ (wherein $R^3$ and $R^4$ independently represent hydrogen or lower alkyl) and $-NR^3R^4$ (wherein $R^3$ and $R^4$ have the same singificances as defined above); and n is an integer of 1 to 6;

Y represents a member selected from the group consisting of hydrogen, $-NR^{1a}R^{2a}$ (wherein $R^{1a}$ and $R^{2a}$ have the same significances as $R^1$ and $R^2$ described above),

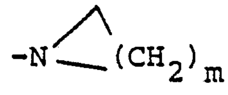

(wherein m represents an integer of 1 to 5),

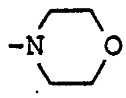 and 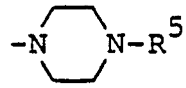

(wherein $R^5$ represents hydrogen, lower alkyl, lower alkanoyl or benzyl);

Q represents a member selected from the group consisting of lower alkyl, hydroxyl, lower alkoxyl, lower alkanoyloxy, halogen and $-O(CH_2)_\ell X^1$ (wherein $X^1$ has the same significance as X described above and $\ell$ represents an integer of 1 to 6) and Qs are the same or different from each other;

q represents an integer of 0 to 4; and

Z represents a member selected from the group consisting of hydrogen, hydroxyl, lower alkoxyl and lower alkanoyloxy.

## DETAILED DESCRIPTION OF THE INVENTION

In the definitions of the groups in formula (I), the alkyl moiety in the lower alkyl, lower alkoxyl, lower alkylthio, lower alkoxycarbonyl and lower alkylsulfonyloxy groups includes a straight-chain or branched alkyl group having 1 to 6 carbon atoms, for example, methyl, ethyl, propyl, isopropyl, butyl, isobutyl, sec-butyl, tert-butyl, pentyl and hexyl. The cycloalkyl moiety in the cycloalkyl and cycloalkyloxy groups includes a cycloalkyl group having 3 to 6 carbon atoms, for example, cyclopropyl, cyclobutyl, cyclopentyl and cyclohexyl. The alkanoyl moiety in the lower alkanoyl, lower alkanoyloxy and lower alkanoylamino groups includes a straight-chain or branched alkanoyl group having 1 to 6 carbon atoms, for example, formyl, acetyl, propionyl, isopropionyl, butyryl, isobutyryl, pivaloyl, valeryl, hexanoyl and trifluoroacetyl. The aryl moiety in the optionally substituted arylsulfonyloxy group includes phenyl, tolyl, etc. and the halogen includes fluorine, chlorine, bromine and iodine.

The pharmaceutically acceptable salts of Compounds (I) include pharmaceutically acceptable acid addition salts which are exemplified by inorganic acid addition salts such as hydrochloride, hydrobromide, sulfate and phosphate, and organic acid addition salts such as oxalate, acetate, malonate, succinate, fumarate, maleate, tartrate and citrate.

The processes for preparing Compounds (I) are described below.

In the processes shown below, in cases where the defined groups change under the conditions of embodiments given or are inadequate for conducting the processes, the groups may be treated in a conventional manner in organic synthetic chemistry, for example, subjected to protection of functional groups or removal of protective groups so that the processes can easily be practiced.

Compounds (I) can be prepared according to the following reaction steps.

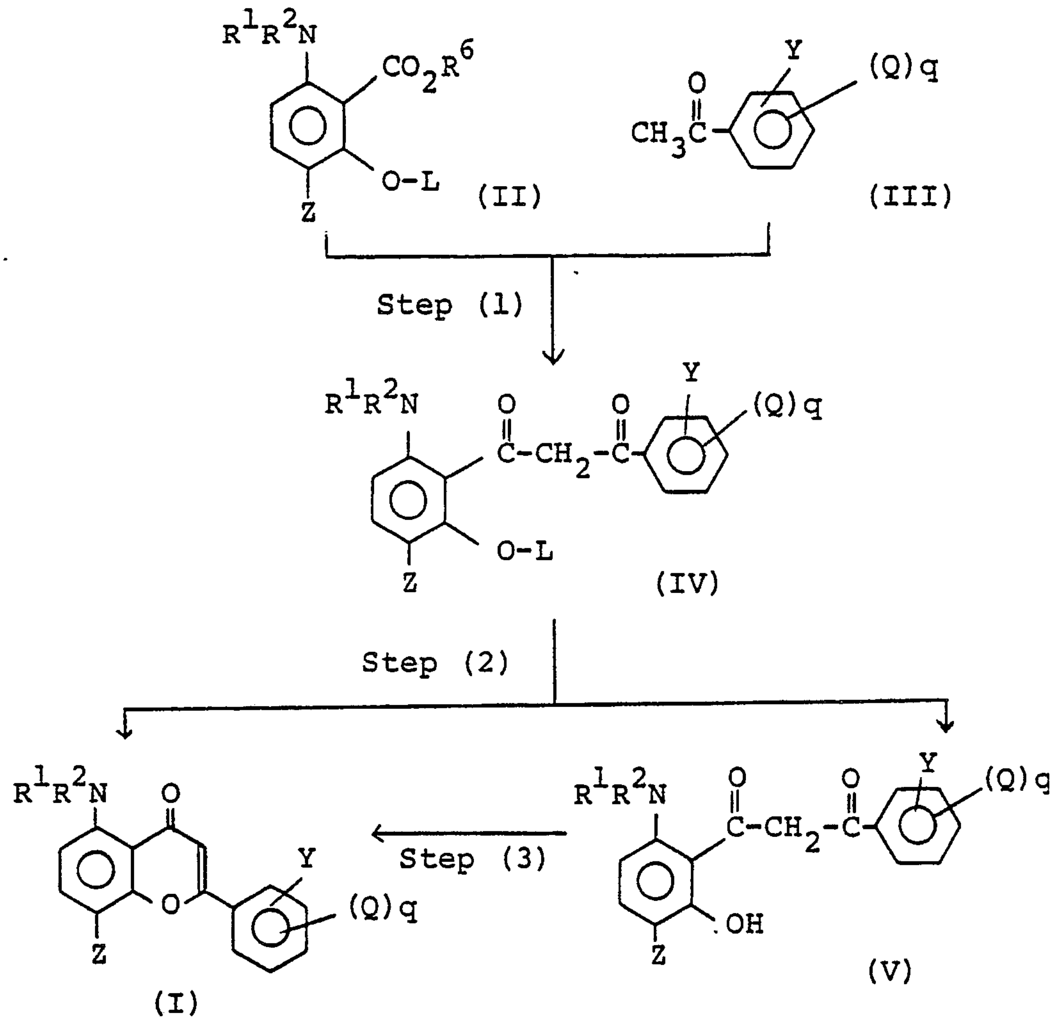


(II) (III) (IV) (I) (V)

In the above formulae, $R^6$ represents lower alkyl; L represents a protective group for hydroxyl; and $R^1$, $R^2$, Q, Y, Z and q have the same significances as defined above.

The lower alkyl shown by $R^6$ has the same significance as that in formula (I) described above. As the protective group for hydroxyl shown by L, protective groups generally known to be those for phenols can be used, and tetrahydropyranyl, methoxymethyl, etc. which are removable by a weak acid are preferably used.

Step (1)

Compound (IV) can be obtained by condensing Compound (II) with an equivalent amount of Compound (III) in an inert solvent in the presence of a base in an amount of 1 to 5 equivalents. Examples of the base are sodium hydride, potassium hydride, sodium methoxide, sodium ethoxide and potassium tert-butoxide. As the suitable inert solvent, ethers such as tetrahydrofuran, dioxane and dimethoxyethane, alcohols such as methanol, ethanol and tert-butanol, dimethylformamide, etc. can be used singly or in combination. The reaction is carried out usually with heating, preferably at a temperature of 40 °C to a boiling point of the solvent used and is completed in 1 to 12 hours.

Compounds (II) which are the starting compounds can be synthesized in accordance with the known method (Japanese Published Unexamined Patent Application No. 78/86).

Step (2)

Subsequently, Compound (IV) obtained in Step (1) is subjected to a reaction for removing the protective group, whereby Compound (I) and/or Compound (V) is formed depending upon the kind of Compound (IV) or the reaction conditions.

In cases, for example, where tetrahydropyranyl or methoxymethyl mentioned above is used as the protective group, the reaction for removing the protective group of Compound (IV) is carried out using an acid or an acid diluted with water in an inert solvent such as a lower alcohol, e.g., methanol, ethanol, propanol and isopropanol, an ether, e.g., dioxane and tetrahydrofuran, and dimethylformamide or in a mixture of the inert solvent and water at a temperature of 0 to 50° C usually for 0.5 to 10 hours. Examples of the acid include hydrochloric acid, sulfuric acid, acetic acid, trifluoroacetic acid, formic acid and oxalic acid. A preferred concentration of the acid in the solvent is 0.1 to 2 N.

Step (3)

Cyclization of Compound (V) into Compound (I) can be carried out using an acidic resin (for example, Amberlist 15: Rohm & Haas Co.) in accordance with the known method for synthesis of flavones [Bull. Chem. Soc. Jap., 60, 1919 (1987))].

The reaction is carried out in an inert solvent with heating, using the acidic resin in an amount of 0.2 -5 times that of Compound (V) by weight. As the inert solvent, benzene, toluene, chloroform, acetonitrile, ethanol, isopropanol, dioxane, tetrahydrofuran, dimethylformamide, etc. can be used singly or in combination. The reaction is carried out at a temperature of 60° C to a boiling point of the solvent and is usually completed in 2 to 20 hours.

Alternatively, the cyclization of Compound (V) into Compound (I) can be carried out under similar conditions as in Step (2) using the same acids as used in Step (2) optionally diluted with water.

Further, Compounds (I) can also be prepared according to the following reaction steps.

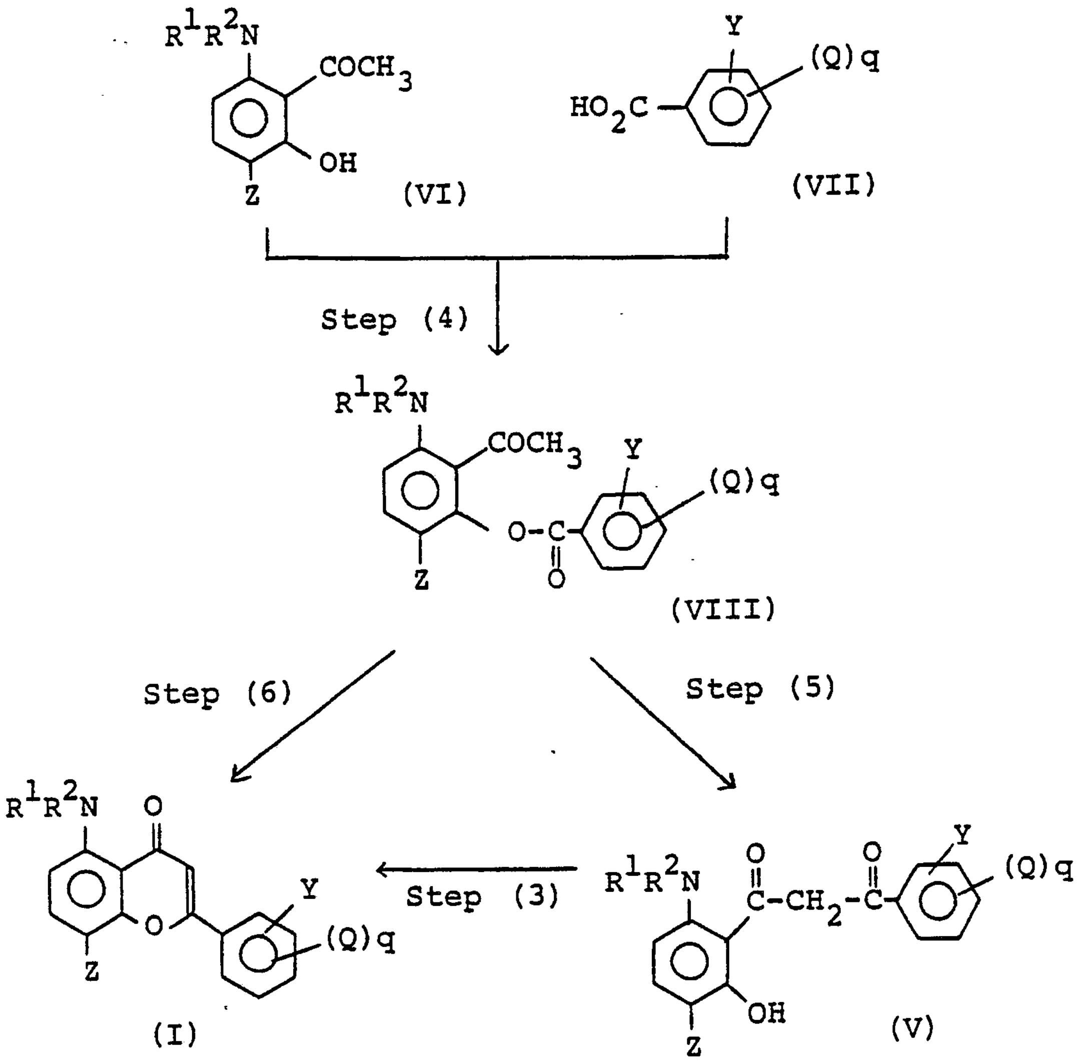

In the above formulae, $R^1$, $R^2$, Q, Y, Z and q have the same significances as defined above.

Step (4)

Compound (VIII) can be obtained by condensing Compound (VI) with a reactive derivative of Compound (VII) such as an acid chloride or an acid anhydride thereof in an amount of 1 to 5 equivalents based on Compound (VI) in an inert solvent in the presence of a base in an amount of 1 to 5 equivalents based on Compound (VI). Alternatively, Compound (VI) may be allowed to react with Compound (VII) in an amount of 1 to 5 equivalents in an inert solvent in the presence of a suitable condensing agent. As the base, sodium hydride, potassium hydride, potassium tert-butoxide, triethylamine, pyridine, quinoline, etc. may be used. As the inert solvent, tetrahydrofuran, dioxane, dimethylformamide, dimethoxyethane, dichloromethane, chloroform, pyridine, etc. may be used singly or in combination. Examples of the condensing agent include trifluoroacetic acid anhydride, dicyclohexylcarbodiimide and 2-chloro-1-methylpyridinium iodide. The reaction is carried out usually at a temperature of 0 °C to a boiling point of the solvent and is completed in 1 to 12 hours.

Step (5)

Then, Compound (VIII) is subjected to rearrangement in an inert solvent in the presence of a base to give Compound (V). Examples of the base are sodium hydride, potassium hydride, sodium hydroxide, potassium hydroxide and potassium carbonate. As the inert solvent, tetrahydrofuran, dimethylsulfoxide, dioxane, diethyl ether, dichloromethane, dimethylformamide, dimethoxyethane, etc. may be used singly or in combination. The reaction is carried out usually at a temperature of 0 °C to a boiling point of the solvent and is completed in 1 to 12 hours.

Compound (I) can be obtained from Compound (V) under the same conditions as described in Step (3).

Step (6)

Compound (VIII) obtained in Step (4) is subjected to reaction in an inert solvent in the presence or absence of a base to give Compound (I). Examples of the base are sodium hydride, potassium hydride, potassium carbonate, sodium hydroxide and potassium hydroxide. As the inert solvent, tetrahydrofuran, dimethylsulfoxide, dioxane, dimethylformamide, dimethoxyethane, diglyme, dichloroethane, tetrachloroethane, ethylene glycol methyl ether, ethylene glycol, etc. may be used singly or in combination. The reaction is carried out usually at a temperature of 100 °C to 200 °C and is completed in 1 to 24 hours. Depending upon the solvent, the reaction may be carried out under pressure.

Some of Compounds (I) obtained herein may be used as synthetic intermediates to further give novel derivatives (I).

For example, Compounds (I) having an amino group at the 5-position ($R^1$ = $R^2$ = H) [hereinafter referred to as Compounds (Ia)] can be obtained by hydrolyzing Compounds (I) wherein $R^1$ and/or $R^2$ is a lower alkanoyl group in a conventional manner. In the case, for example, of Compound (I) wherein $R^1$ or $R^2$ is a pivaloyl group, the compound is allowed to react with hydrochloric acid in a lower alcohol or in an inert solvent miscible with water at a temperature of 0 °C to a boiling point of the solvent. The reaction is completed usually in 0.1 to 10 hours. As the lower alcohol, those mentioned in Step (2) can be used. Examples of the inert solvent miscible with water are tetrahydrofuran, dioxane, dimethoxyethane, acetic acid and dimethylformamide. For the reaction, 0.1 to 10 N hydrochloric acid is used in an amount of 0.1 to 1 times that of the solvent by volume.

Compounds (I) wherein $R^{1a}$ and/or $R^{2a}$ in $-NR^{1a}R^{2a}$ represented by Y is lower alkyl, benzyl or $-(CH_2)_nX^a$ (wherein $X^a$ represents lower alkoxyl, lower alkylthio, halogen, carboxyl or $-NR^3R^4$ in the definition of X; and n has the same significance as defined above) [hereinafter referred to as Compounds (Ib)] can be obtained by allowing Compounds (I) wherein Y is amino optionally protected with acetyl, trifluoroacetyl, etc. to react with lower alkyl halide, benzyl halide or Compounds (IX) represented by the formula:

$Hal - (CH_2)_nX^a$ (IX)

(wherein Hal represents halogen such as chlorine, bromine or iodine, and $X^a$ and n have the same significances as defined above) or, when appropriate, acid addition salts thereof (for example, hydrochloride, hydrobromide, sulfate, acetate, trifluoroacetate and p-toluenesulfonate; hereinafter acid addition salts

mean the same) in an inert solvent at room temperature to 120°C for 1 to 24 hours in the presence of a base in an amount of one equivalent to excess based on the starting Compounds (I). The halogen in the lower alkyl halide and benzyl halide has the same significance as Haℓ mentioned above. In the case where Haℓ is chlorine or bromine, the use of sodium iodide or potassium iodide in an amount of 0.05 to one equivalent based on the starting Compounds (I) as a catalyst sometimes accelerates the reaction. Examples of the base include sodium hydride, potassium hydride, sodium carbonate and potassium carbonate. Examples of the inert solvent are dimethylformamide, dimethylsulfoxide, tetrahydrofuran, dioxane and dichloromethane.

When Y has the protective group such as acetyl or trifluoroacetyl mentioned above, the product is treated with hydrochloric acid in a lower alcohol or in an inert solvent miscible with water at a temperature of 0°C to a boiling point of the solvent usually for 0.1 to 10 hours, whereby the deacetylated or detrifluoroacetylated product can be obtained. As the lower alcohol, those mentioned in Step (2) can be used. Examples of the inert solvent miscible with water include tetrahydrofuran, dioxane, dimethylformamide and acetic acid. For the reaction, 0.1 to 10 N hydrochloric acid is used in an amount of 0.1 to 1 times that of the solvent by volume.

Compounds (I) wherein $R^{1a}$ and/or $R^{2a}$ in $-NR^{1a}R^{2a}$ represented by Y is $-(CH_2)_nX^b$ (wherein $X^b$ represents chlorine, bromine or iodine and n has the same significance as defined above) [hereinafter referred to as Compounds (Ic)] can be obtained by allowing Compounds (I) wherein $R^{1a}$ and/or $R^{2a}$ in $-NR^{1a}R^{2a}$ represented by Y is $-(CH_2)_nOH$ (wherein n has the same significance as defined above) [hereinafter referred to as Compounds (Id)] to react with thionyl halide or phosphorus halide in an amount of one equivalent to excess based on Compounds (Id) in the absence of a solvent or in an inert solvent at a temperature of 0 to 100°C for 1 to 24 hours. Examples of the thionyl halide include thionyl chloride and thionyl bromide, and examples of the phosphorus halide include phosphorus trichloride, phosphorus pentachloride, phosphorus tribromide, phosphorus pentabromide and phosphorus triiodide. As the inert solvent, dimethylformamide, tetrahydrofuran, diethyl ether, dioxane, chloroform, dichloromethane, pyridine, etc. can be used singly or in combination.

Alternatively, Compounds (Ic) can be obtained by allowing Compounds (I) wherein $R^{1a}$ and/or $R^{2a}$ in $-NR^{1a}R^{2a}$ represented by Y is $-(CH_2)_nX^c$ (wherein $X^c$ represents lower alkylsulfonyloxy, trifluoromethanesulfonyloxy or optionally substituted arylsulfonyloxy in the definition of X; and n has the same significance as defined above) [hereinafter referred to as Compounds (Ie)] to react with alkali halide or tetraalkylammonium halide in an amount of 1 to 5 equivalents based on Compounds (Ie) in an inert solvent at a temperature of 0 to 100°C for 1 to 24 hours. Examples of the alkali halide include lithium chloride, lithium bromide, magnesium bromide, calcium bromide and sodium iodide. Examples of the tetraalkylammonium halide are tetrabutylammonium chloride, tetrabutylammonium bromide, tetrabutylammonium iodide and tetrabutylammonium fluoride. As the inert solvent, dimethylformamide, dimethylsulfoxide, ethanol, methanol, diethyl ether, tetrahydrofuran, dioxane, etc. can be used singly or in combination.

Compounds (Ie) can be obtained by allowing Compounds (Id) to react with sulfonyl halide in an inert solvent in the presence of a base at a temperature of 0 to 100°C for 0.5 to 12 hours. The sulfonyl halide and the base are respectively used in an amount of 1 to 5 equivalents based on Compounds (Id). Examples of the base are sodium hydride, potassium hydride, sodium hydroxide, potassium hydroxide, sodium carbonate, triethylamine and pyridine. Examples of the sulfonyl halide are p-toluenesulfonyl chloride, methanesulfonyl chloride, trifluoromethanesulfonyl chloride and benzenesulfonyl chloride. As the inert solvent, dimethylformamide, dimethylsulfoxide, tetrahydrofuran, dioxane, dichloromethane, diethyl ether, etc. can be used singly or in combination.

Compounds (I) wherein $R^{1a}$ and/or $R^{2a}$ in $-NR^{1a}R^{2a}$ represented by Y is $-(CH_2)_nX^d$ (wherein $X^d$ represents lower alkoxyl, lower alkylthio or $-NR^3R^4$ in the definition of X; and n has the same significance as defined above) [hereinafter referred to as Compounds (If)] can be obtained by allowing Compounds (Ic) or Compounds (Ie) with the corresponding lower alcohol, lower alkylthiol or amine represented by $NHR^3R^4$ (wherein $R^3$ and $R^4$ have the same significances as defined above) in an inert solvent at 0 to 100°C for 1 to 24 hours, optionally in the presence of a base. The lower alcohol, lower alkylthiol or amine and the base are respectively used in an amount of 1 to 5 equivalents based on Compounds (Ic) or (Ie). Examples of the base are sodium hydride, potassium hydride, sodium hydroxide, potassium hydroxide, sodium carbonate, triethylamine and pyridine. As the inert solvent, dimethylformamide, dimethylsulfoxide, tetrahydrofuran, dioxane, dichloromethane, diethyl ether, etc. can be used singly or in combination.

Compounds (If) can also be obtained by allowing Compounds (Id) or Compounds (I) wherein $R^{1a}$ and/or $R^{2a}$ in $-NR^{1a}R^{2a}$ represented by Y is $-(CH_2)_nX^e$ (wherein $X^e$ represents amino or mercapto in the definition of X; and n has the same significance as defined above) to react with the corresponding lower alkyl halide or sulfonic acid ester of lower alkyl alcohol in an inert solvent at 0 to 100°C for 1 to 24 hours, optionally in the

presence of a base. The lower alkyl halide or sulfonic acid ester of lower alkyl alcohol and the base are respectively used in an amount of 1 to 5 equivalents based on the starting Compounds (I). Examples of the sulfonic acid moiety in the sulfonic acid ester are p-toluenesulfonic acid and methanesulfonic acid. Examples of the base are sodium hydride, potassium hydride, sodium carbonate, sodium hydroxide, potassium hydroxide, triethylamine and pyridine. As the inert solvent, dimethylformamide, dimethylsulfoxide, dichloromethane, dichloroethane, tetrahydrofuran, dioxane, etc. can be used singly or in combination.

Compounds (I) wherein $R^{1a}$ and/or $R^{2a}$ in $-NR^{1a}R^{2a}$ represented by Y is $-(CH_2)_nCO_2R^{3a}$ (wherein $R^{3a}$ represents lower alkyl in the definition of $R^3$ and n has the same significance as defined above) or $-(CH_2)_nCONR^3R^4$ (wherein $R^3$, $R^4$ and n have the same significances as defined above) can be obtained by subjecting Compounds (I) wherein $R^{1a}$ and/or $R^{2a}$ in $-NR^{1a}R^{2a}$ represented by Y is $-(CH_2)_nCOOH$ (wherein n has the same significance as defined above) and the corresponding lower alcohol, lower alkyl halide or amine represented by $HNR^3R^4$ (wherein $R^3$ and $R^4$ have the same significances as defined above) to esterification or amidation reaction generally used in organic synthetic chemistry.

Compounds (I) wherein Y is

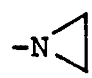

can be obtained by treating Compounds (I) wherein Y represents $-NH(CH_2)_2X^{bc}$ (wherein $X^{bc}$ has the same significance as $X^b$ or $X^c$ mentioned above) with a base at 0 to 100° C for 1 to 24 hours. As the base, sodium hydride, potassium hydride, sodium carbonate, sodium hydroxide, potassium hydroxide, etc. may be used in an amount of 1 to 5 equivalents based on the starting Compounds (I). As the inert solvent, dimethylformamide, dimethylsulfoxide, tetrahydrofuran, dioxane, dichloromethane, dichloroethane, etc. can be used singly or in combination.

Compounds (I) wherein Y is $-NH(CH_2)_2X^b$ (wherein $X^b$ has the same significance as defined above) can be obtained by allowing the above-mentioned Compounds (I) wherein Y is

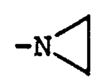

to react with hydrogen halide in an inert solvent with heating under reflux. As the hydrogen halide, hydrogen chloride, hydrochloric acid, hydrobromic acid and hydriodic acid may be used in an amount of one equivalent to excess based on the starting Compounds (I). As the inert solvent, methanol, ethanol, dioxane, etc. can be used singly or in combination.

Compounds (I) wherein $R^1$ and/or $R^2$ is lower alkyl, benzyl or $-(CH_2)_nX^a$ (wherein $X^a$ and n have the same significances as defined above) can be obtained by allowing Compounds (Ia) to react with lower alkyl halide, benzyl halide or Compounds (IX) or acid addition salts thereof under similar conditions as in the preparation of Compounds (Ib) described above.

Compounds (I) wherein one of $R^1$ and $R^2$ is amino and the other is hydrogen [hereinafter referred to as Compounds (Ig)] can be obtained by diazotizing Compounds (Ia) with sodium nitrite in an acidic solvent under cooling and subsequently reducing the resulting diazo compounds with a suitable reducing agent. Sodium nitrite is used in an amount of 1 to 2 equivalents based on Compounds (Ia). As the acidic solvent, acetic acid, hydrochloric acid, sulfuric acid, etc. can be used singly or in combination with water. Examples of the suitable reducing agent include stannous chloride, zinc powder and iron powder, which are preferably used in an amount of 1 to 5 equivalents based on the diazo compounds. The diazotization is carried out at -15 to 5° C for 0.1 to 2 hours and the reduction is carried out at 0° C to room temperature for 1 to 10 hours.

Compounds (I) wherein Y is $-NHNH_2$ can be obtained from Compounds (I) wherein Y is amino under similar conditions as in the preparation of Compounds (Ig) described above.

Compounds (I) wherein $R^1$ and/or $R^2$ is lower alkanoyl or lower alkanoylamino can be obtained by allowing the corresponding Compounds (Ia) or (Ig) with the corresponding lower aliphatic carboxylic acid or reactive derivatives of the carboxylic acid such as acid anhydride, acid chloride and acid bromide, optionally in the presence of a base. Examples of the base are triethylamine, pyridine, dimethylaminopyridine, sodium carbonate and potassium carbonate. As the reaction solvent, dimethylformamide, dimethylsulfoxide, tetrahydrofuran, dioxane, etc. can be used. Further, the carboxylic acid or reactive derivatives thereof can also be used as the solvent. The reaction is carried out at a temperature

ranging from that under ice cooling to a boiling point of the solvent or carboxylic acid or reactive derivatives thereof used for 0.5 to 10 hours.

Compounds (I) wherein $R^{1a}$ and/or $R^{2a}$ in $-NR^{1a}R^{2a}$ represented by Y is lower alkanoyl or lower alkanoylamino can be obtained from Compounds (I) wherein the corresponding group is amino under similar conditions as in the reaction described above.

Further, Compounds (I) wherein Q or Z is lower alkanoyloxy can be obtained by allowing Compounds (I) wherein the corresponding group is hydroxyl to react with the corresponding lower aliphatic carboxylic acid or reactive derivatives thereof under similar conditions as in the reaction described above.

Compounds (I) wherein Q or Z is lower alkoxyl can be obtained by allowing Compounds (I) wherein the corresponding group is hydroxyl to react with the corresponding lower alkyl halide in the presence of a base. As the base, triethylamine, pyridine, dimethylaminopyridine, sodium carbonate, potassium carbonate, etc. may be used in an amount of 1 to 5 equivalents based on the starting Compounds (I). As the reaction solvent, dimethylformamide, dimethylsulfoxide, tetrahydrofuran, dioxane, etc. can be used. Further, the lower alkyl halide can also be used as the solvent. The reaction is carried out at a temperature ranging from that under ice cooling to a boiling point of the solvent or lower alkyl halide used for 0.5 to 10 hours.

Compounds (I) wherein Q is $-O(CH_2)_\ell X^a$ (wherein $X^a$ and $\ell$ have the same significances as defined above) can be obtained by allowing Compounds (I) wherein the corresponding group is hydroxyl to react with Compounds (IX) under similar conditions as in the reaction described above.

Compounds (I) wherein $R^1$ or $R^2$, or $R^{1a}$ or $R^{2a}$ in $-NR^{1a}R^{2a}$ represented by Y is methyl can be obtained by allowing Compounds (Ia) or Compounds (I) wherein Y is amino to react with formaldehyde in an inert solvent, followed by reduction. As the formaldehyde, a formalin aqueous solution, paraformaldehyde, etc. may be used. Examples of the reducing agent which is used for the reduction include sodium borohydride, sodium cyanoborohydride, lithium cyanoborohydride and lithium aluminum hydride. As the inert solvent, dimethylformamide, dimethylsulfoxide, dioxane, tetrahydrofuran, acetic acid, water, etc. can be used singly or in combination. The reaction is carried out usually at 0 to 100 °C and is completed in 1 to 24 hours.

Compounds (I) having desired functional groups at desired positions can be obtained by properly combining the above-described processes, etc.

The intermediates and the final products in the respective processes described above can be isolated and purified by purification methods conventionally used in organic synthetic chemistry, for example, filtration, extraction, washing, drying, concentration, recrystallization and various kinds of chromatography. In some cases, the intermediates can be subjected to the subsequent reaction without any particular purification.

In the case where a salt of Compound (I) is desired and it is produced in the form of the desired salt, it can be subjected to purification as such. In the case where Compound (I) is produced in the free state and its salt is desired, it can be converted into its salt in a conventional manner.

Compounds (I) and pharmaceutically acceptable salts thereof sometimes exist in the form of an addition product with water or with a solvent. Such addition products are also included within the scope of the present invention.

Specific examples of Compounds (I) which can be obtained by the present invention are shown in Table 1.

Table 1

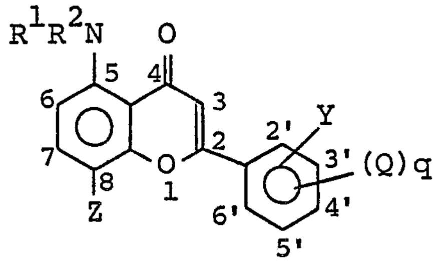

| Compound No. | $NR^1R^2$ | Y | (Q)q | Z |
|---|---|---|---|---|
| 1 | $NHCOC(CH_3)_3$ | $4'-NHCOC(CH_3)_3$ | (q=0) | H |
| 2 | $NH_2$ | $4'-NH_2$ | (q=0) | H |
| 3 | $NH_2$ | $4'-NHCH_2CH_2N(CH_3)_2$ | (q=0) | H |
| 4 | $NHCOC(CH_3)_3$ | $3'-NHCOC(CH_3)_3$ | (q=0) | H |
| 5 | $NH_2$ | $3'-NH_2$ | (q=0) | H |
| 6 | $NHCOCH_3$ | $4'-NHCOCH_3$ | (q=0) | H |
| 7 | $NHCH_3$ | $4'-N(CH_3)_2$ | (q=0) | H |
| 8 | $NH_2$ | $4'-N(CH_3)_2$ | (q=0) | H |
| 9 | $NH_2$ | $4'-NHCH_3$ | (q=0) | H |
| 10 | $NH_2$ | 4'- 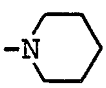 | (q=0) | H |
| 11 | $NH_2$ | 4'- 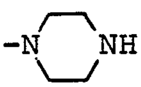 | (q=0) | H |
| 12 | $NHCOCH_3$ | $4'-N(CH_3)_2$ | (q=0) | H |
| 13 | $NH_2$ | $4'-NHCOCH_3$ | (q=0) | H |
| 14 | $NH_2$ | $4'-NH_2$ | 2'-OH | H |
| 15 | $NH_2$ | $4'-NHCH_2CH_3$ | (q=0) | H |
| 16 | $NHCOC(CH_3)_3$ | $4'-NCH(CH_3)_2$<br>$COCH_3$ | (q=0) | H |

| Compound No. | $NR^1R^2$ | Y | (Q)q | Z |
|---|---|---|---|---|
| 17 | $NH_2$ | 4'-$N(COCH_3)CH(CH_3)_2$ | (q=0) | H |
| 18 | $NH_2$ | 4'-$NHCH(CH_3)_2$ | (q=0) | H |
| 19 | $NH_2$ | 4'-$N(CH_2CH_2OH)_2$ | (q=0) | H |
| 20 | $NHCOC(CH_3)_3$ | 4'-$N(CH_2CH_2Cl)_2$ | (q=0) | H |
| 21 | $NH_2$ | 4'-$N(CH_2CH_2Cl)_2$ | (q=0) | H |
| 22 | $NH_2$ | 4'-$NHCH_2CH_2CH_3$ | (q=0) | H |
| 23 | $NH_2$ | 4'-$NH(CH_2)_3CH_3$ | (q=0) | H |
| 24 | $NH_2$ | 4'-NHBzl | (q=0) | H |
| 25 | $NH_2$ | 4'-$NHCH_2CO_2H$ | (q=0) | H |
| 26 | $NH_2$ | 4'-$NHCH_2CO_2CH_2CH_3$ | (q=0) | H |
| 27 | $NH_2$ | 4'-$NHCH_2CO_2CH_3$ | (q=0) | H |
| 28 | $NH_2$ | 4'-$NHCH_2CH_2OH$ | (q=0) | H |
| 29 | $NH_2$ | 4'-$NH_2$ | 3'-OH | H |
| 30 | $NHCOC(CH_3)_3$ | 4'-$NHCOC(CH_3)_3$ | 3'-F | H |
| 31 | $NH_2$ | 4'-$NH_2$ | 3'-F | H |
| 32 | $NHCOC(CH_3)_3$ | 4'-$NHCOC(CH_3)_3$ | 3'-F, 5'-F | H |
| 33 | $NH_2$ | 4'-$NH_2$ | 3'-F, 5'-F | H |
| 34 | $NHCOC(CH_3)_3$ | 4'-$NHCH_2CH_2OCH_3$ | (q=0) | H |
| 35 | $NH_2$ | 4'-$NHCH_2CH_2OCH_3$ | (q=0) | H |
| 36 | $NH_2$ | 4'-$NH_2$ | (q=0) | $OCH_3$ |
| 37 | $NH_2$ | 4'-$NHCH_3$ | (q=0) | $OCH_3$ |
| 38 | $NH_2$ | 4'-$NHCH_2CH_2Br$ | (q=0) | H |

| Compound No. | $NR^1R^2$ | Y | (Q)q | Z |
|---|---|---|---|---|
| 39 | $NH_2$ | 4'-$NHCH_2CH_2Cl$ | (q=0) | H |
| 40 | $NH_2$ | 4'-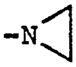 | (q=0) | H |
| 41 | $NHCOC(CH_3)_3$ | 4'-$NHCOCH_3$ | (q=0) | H |
| 42 | $NHCOC(CH_3)_3$ | 4'-$NHCH_2CH_2OTs$ | (q=0) | H |
| 43 | $NH_2$ | 4'-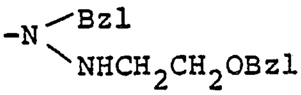 | (q=0) | H |
| 44 | $NH_2$ | 4'-$NHCH_2CH_2OBzl$ | (q=0) | H |
| 45 | $NH_2$ | 4'-$NHCH_2CH_2OC_2H_5$ | (q=0) | H |
| 46 | $NHCOCH_3$ | 4'-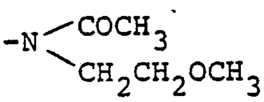 | (q=0) | H |
| 47 | $NH_2$ | 4'-$NH_2$ | (q=0) | OH |
| 48 | $NHCOC(CH_3)_3$ | H | 4'-OH | H |
| 49 | $NH_2$ | H | 4'-OH | H |
| 50 | $NH_2$ | H | 3'-OH | H |
| 51 | $NH_2$ | H | 3'-OH, 5'-OH | H |
| 52 | $NH_2$ | H | 4'-$OCH_2CH_2N(CH_3)_2$ | H |
| 53 | $NH_2$ | H | 3'-$OCH_2CH_2N(CH_3)_2$ | H |
| 54 | $NHCOC(CH_3)_3$ | H | 3'-$OCH_3$, 4'-$OCH_3$ | $OCH_3$ |
| 55 | $NH_2$ | H | 3'-$OCH_3$, 4'-$OCH_3$ | $OCH_3$ |
| 56 | $NH_2$ | H | (q=0) | $OCH_3$ |
| 57 | $NH_2$ | H | 3'-$OCH_3$ | $OCH_3$ |
| 58 | $NH_2$ | H | 4'-$OCH_3$ | $OCH_3$ |
| 59 | $NH_2$ | H | 3'-$OCH_3$ | H |
| 60 | $NH_2$ | H | (q=0) | H |

| Compound No. | $NR^1R^2$ | Y | (Q)q | Z |
|---|---|---|---|---|
| 61 | $NHNH_2$ | H | (q=0) | H |
| 62 | $NHCH_2CH_2N(CH_3)_2$ | H | (q=0) | H |
| 63 | $NHNHCOCH_3$ | H | (q=0) | H |
| 64 | $NH_2$ | H | 3'-$OCH_3$, 4'-$OCH_3$ | H |
| 65 | $NH_2$ | H | 3'-$OCH_3$, 4'-OH, 5'-$OCH_3$ | H |
| 66 | $NH_2$ | H | 2'-$OCH_3$, 4'-$OCH_3$ | H |
| 67 | $NH_2$ | H | 3'-$OCH_3$, 5'-$OCH_3$ | H |
| 68 | $NH_2$ | H | 2'-OH, 4'-OH | H |
| 69 | $NH_2$ | H | 4'-$OCH_3$ | H |
| 70 | $NH_2$ | H | 4'-Br | H |

Ts reprsents $SO_2$-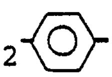-$CH_3$.

Bzl represents $CH_2$-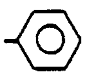.

The anticellular activity of the compounds obtained by the present invention is shown below referring to test examples.

Test Example 1

Growth inhibition test on human colon adenocarcinoma (WiDr):

WiDr cells (6 x$10^4$ cells/ml) prepared in a medium comprising MEM medium (Nissui Pharmaceutical Co., Ltd.), 2 mM glutamine, 10% fetal calf serum and 1% non-essential amino acids kit (Dainippon Pharmaceutical Co., Ltd.) (hereinafter referred to as Medium A) were put into wells of a 96-well microtiter plate in an amount of 0.1 ml/well. The cells on the plate were incubated in a $CO_2$-incubator at 37° C for 20 hours, and 0.05 ml of a sample containing a test compound and appropriately diluted with Medium A was added to each well. The cells were further incubated in the $CO_2$-incubator at 37° C for 72 hours. After the culture supernatant was removed, Medium A containing 0.02% Neutral Red was added to the residue in an amount of 0.1 ml/well, followed by incubation at 37° C for one hour in the $CO_2$-incubator, whereby the cells were stained. The culture supernatant was removed and the residue was washed once with physiological saline solution. The pigment was extracted with 0.001N-HCl /30%-ethanol, and the absorbance was determined at 550 nm with a microplate reader. The absorbance determined for intact cells was compared with that for the cells treated with the test compound at a known concentration, and the concentration of the test compound for inhibiting 50% growth of the cells ($IC_{50}$) was calculated. The results obtained are shown in Table 2-1.

Table 2-1

| Compound No. | $IC_{50}$ (μM) |
|---|---|
| 1 | 16 |
| 2 | 26 |
| 3 | 10 |
| 7 | 28 |
| 9 | 20 |
| 11 | 6.5 |
| 16 | 5.9 |
| 18 | 8.0 |
| 21 | 4.8 |
| 40 | 8.9 |
| 45 | 15 |
| 52 | 17 |
| 55 | 23 |
| 56 | 33 |
| 57 | 24 |
| 58 | 26 |
| 59 | 30 |
| 62 | 12 |
| 68 | 39 |
| Flavone acetic acid | 54 |
| Quercetin | 53 |

Test Example 2

Growth inhibition test on human breast adenocarcinoma (MCF-7):

MCF-7 cells (5 x$10^4$ cells/ml) prepared in a medium comprising RPMI1640 medium, 10% fetal calf serum, $10^{-8}$M estradiol (Sigma Fine Chemical Inc.), 100 units/ml penicillin and 100 μg/ml streptomycin (hereinafter referred to as Medium B) were put into wells of a 96-well microtiter plate in an amount of 0.1 ml/well. The cells on the plate were incubated in a $CO_2$-incubator at 37° C for 20 hours, and 0.05 ml of a sample containing a test compound and appropriately diluted with Medium B was added to each well. The cells were further incubated in the $CO_2$-incubator at 37° C for 72 hours. After the culture supernatant was removed, Medium B containing 0.02% Neutral Red was added to the residue in an amount of 0.1 ml/well, followed by incubation at 37° C for one hour in the $CO_2$-incubator, whereby the cells were stained. The subsequent steps were carried out in the same manner as in Test Example 1 and $IC_{50}$ was calculated. The results obtained are shown in Table 2-2.

Table 2-2

| Compound No. | $IC_{50}$ (μM) |
|---|---|
| 7 | 16 |
| 11 | 4.9 |
| 16 | 6.9 |
| 21 | 8.4 |
| 26 | 2.6 |
| 27 | 1.5 |
| 31 | 2.2 |
| 37 | 3.6 |
| 40 | 25 |
| Flavone acetic acid | 100 |
| Quercetin | 26 |

Test Example 3

Growth inhibition test on human curvix epipheloid carcinoma (HeLa $S_3$):

HeLa $S_3$ cells (3 x$10^4$ cells/ml) prepared in a medium comprising MEM medium, 2 mM glutamine and 10% fetal calf serum (hereinafter referred to as Medium C) were put into wells of a 96-well microtiter plate in an amount of 0.1 ml/well. The cells on the plate were incubated in a $CO_2$-incubator at 37° C for 20 hours, and 0.05 ml of a sample containing a test compound and appropriately diluted with Medium C was added to each well. The cells were further incubated in the $CO_2$-incubator at 37° C for 72 hours. After the culture supernatant was removed, Medium C containing 0.02% Neutral Red was added to the residue in an amount of 0.1 ml/well, followed by incubation at 37° C for one hour in the $CO_2$-incubator, whereby the cells were stained. The subsequent steps were carried out in the same manner as in Test Example 1 and $IC_{50}$ was calculated. The results obtained are shown in Table 2-3.

Table 2-3

| Compound No. | $IC_{50}$ (μM) |
|---|---|
| 3 | 20 |
| 4 | 13 |
| 9 | 6.3 |
| 15 | 9.5 |
| 17 | 6.2 |
| 18 | 4.2 |
| 20 | 3.2 |
| 21 | 1.1 |
| 30 | 3.6 |
| 33 | 33 |
| 34 | 6.0 |
| 39 | 8.3 |
| 40 | 2.3 |
| 42 | 4.9 |
| Flavone acetic acid | 50 |
| Quercetin | 10 |

As shown in Tables 2-1 through 2-3, Compounds (I) exhibit anticellular activity and are expected to be useful as therapeutic drugs for various cancers including breast cancer, uterine cancer, endometrial cancer, prostatic cancer and colon cancer.

Certain embodiments of the invention are illustrated in the following examples. The physicochemical properties of Compounds (I) obtained are shown in Table 3.

## Example 1

5-(N-Pivaloyl)amino-2-[4-(N-pivaloyl)amino]phenyl-4H-benzopyran-4-one (Compound 1):

In 8 ml of dioxane was suspended 0.46 g of sodium hydride (60% oil dispersion). A solution of 2.0 g of 2-ethoxycarbonyl-N-pivaloyl-3-(2-tetrahydropyranyl)oxyaniline and 1.26 g of 4-(N-pivaloyl)-aminoacetophenone in 8 ml of dioxane was added dropwise to the suspension with heating under reflux. The mixture was heated under reflux for further one hour. After the reaction mixture was cooled, water was added thereto and the aqueous layer was washed with petroleum ether. The aqueous layer was then extracted with ethyl acetate. The ethyl acetate layer was washed with water, dried over anhydrous sodium sulfate, and concentrated to give 1.89 g of oily 1,3-diketone.

The diketone was subjected to the following cyclization without purification. That is, the 1,3-diketone was dissolved in 15 ml of ethanol and 1.5 ml of concentrated hydrochloric acid was added to the solution, followed by stirring at room temperature for 50 minutes. The reaction mixture was concentrated under reduced pressure and the residue was dissolved in chloroform. The solution was washed successively with a saturated aqueous solution of sodium bicarbonate and water, dried over anhydrous sodium sulfate, and then concentrated. The residue was subjected to silica gel column chromatography to give 1.02 g (yield 67%) of colorless Compound 1.

## Example 2

5-Amino-2-(4-amino)phenyl-4H-benzopyran-4-one (Compound 2):

In 35 ml of ethanol was dissolved 1.02 g of Compound 1 obtained in Example 1, and 17.5 ml of concentrated hydrochloric acid was added to the solution, followed by heating under reflux for 4 hours. The precipitated crystals were separated by filtration and recrystallized from methanol-diethyl ether to give 0.39 g (yield 63%) of the pale yellow hydrochloride of Compound 2.

Example 3

5-Amino-2-[4-(2-dimethylamino)ethylamino]phenyl-4H-benzopyran-4-one (Compound 3):

In 2 ml of dimethylformamide was dissolved 46 mg of Compound 2 obtained in Example 2, and 43.8 mg of sodium hydride (60% oil dispersion) and 78.8 mg of 2-dimethylaminoethyl chloride hydrochloride were added to the solution. The resulting mixture was stirred at 60 to 70 °C for 4 hours. After completion of the reaction, the reaction mixture was concentrated under reduced pressure and the residue was dissolved in chloroform. The solution was washed with water, dried over anhydrous sodium sulfate, and then concentrated. After the residue was partially purified by preparative thin layer chromatography, recrystallization was carried out from isopropyl ether to give 11.0 mg (yield 19%) of pale yellow Compound 3.

Example 4

5-(N-Pivaloyl)amino-2-[3-(N-pivaloyl)amino]phenyl-4H-benzopyran-4-one (Compound 4):

In 8 ml of dioxane was suspended 0.46 g of sodium hydride (60% oil dispersion). A solution of 2.0 g of 2-ethoxycarbonyl-N-pivaloyl-3-(2-tetrahydropyranyl)oxyaniline and 1.26 g of 3-(N-pivaloyl)-aminoacetophenone in 8 ml of dioxane was added dropwise to the suspension with heating under reflux. The mixture was heated under reflux for further 25 minutes. Thereafter, the reaction mixture was treated in a similar manner as in Example 1 to give 2.62 g of an oily substance. The oily substance was dissolved in 15 ml of ethanol without purification and 1.5 ml of concentrated hydrochloric acid was added to the solution, followed by stirring at room temperature for 35 minutes. The reaction mixture was treated in a similar manner as in Example 1 to give 1.03 g (yield 49%) of colorless Compound 4.

Example 5

5-Amino-2-(3-amino)phenyl-4H-benzopyran-4-one (Compound 5):

In 10 ml of ethanol was dissolved 1.03 g of Compound 4 obtained in Example 4, and 5 ml of concentrated hydrochloric acid was added to the solution, followed by heating under reflux for 6.5 hours. The precipitated crystals were separated by filtration and washed successively with cold ethanol and cold diethyl ether to give 0.55 g (yield 88%) of pale yellow Compound 5.

Example 6

5-(N-Acetyl)amino-2-[4-(N-acetyl)amino]phenyl-4H-benzopyran-4-one (Compound 6):

In 1 ml of pyridine was dissolved 200 mg of Compound 2 obtained in Example 2, and 0.85 ml of acetyl chloride was added to the solution. The resulting mixture was stirred at room temperature for 25 minutes, followed by addition of chloroform. The mixture was washed successively with water, a 5% aqueous solution of copper sulfate and a saturated aqueous solution of sodium chloride, dried over anhydrous sodium sulfate, and then concentrated. The residue was triturated with chloroform to give 76 mg (yield 29%) of pale yellow Compound 6.

Example 7

2-(4-Dimethylamino)phenyl-5-methylamino-4H-benzopyran-4-one (Compound 7):

In 8 ml of dimethylformamide was dissolved 100 mg of Compound 2 obtained in Example 2, and 0.5 ml of a 37% aqueous solution of formalin and 118 mg of sodium cyanoborohydride were added to the solution. To the mixture was added 30 µl of acetic acid, followed by stirring at room temperature for 18 hours. After the formed insoluble matters were removed, the reaction mixture was concentrated under reduced pressure. The residue was purified by preparative thin layer chromatography (developing solvent; chloroform : acetone = 20 : 1) to give 5.2 mg (yield 4%) of Compound 7.

Example 8

5-Amino-2-(4-dimethylamino)phenyl-4H-benzopyran-4-one (Compound 8):

Sodium hydride (60% oil dispersion) (114 mg), 500 mg of 2-ethoxycarbonyl-N-pivaloyl-3-(2-tetrahydropyranyl)oxyaniline and 233 mg of 4-dimethylaminoacetophenone were treated in a similar manner as in Example 1. Silica gel column chromatography gave 434 mg of an oily product.

The oily product (384 mg) was cyclized in a similar manner as in Example 1 to give 214 mg (yield 72%) of 2-(4-dimethylamino)phenyl-5-(N-pivaloyl)amino-4H-benzopyran-4-one.

The obtained compound (214 mg) was then treated in a similar manner as in Example 2, followed by silica gel column chromatography. Subsequent recrystallization from chloroform-hexane gave 126 mg (yield 77%) of Compound 8.

Example 9

5-Amino-2-(4-methylamino)phenyl-4H-benzopyran-4-one (Compound 9):

Sodium hydride (60% oil dispersion) (159 mg), 696 mg of 2-ethoxycarbonyl-N-pivaloyl-3-(2-tetrahydropyranyl)oxyaniline and 297 mg of 4-methylaminoacetophenone were treated in a similar manner as in Example 1. Silica gel column chromatography gave 470 mg of an oily product.

The oily product (534 mg) was cyclized in a similar manner as in Example 1. Recrystallization from chloroform-hexane gave 171 mg (yield 42%) of 2-(4-methylamino)phenyl-5-(N-pivaloyl)amino-4H-benzopyran-4-one.

The obtained compound (150 mg) was then treated in a similar manner as in Example 2, followed by silica gel column chromatography. Subsequent recrystallization from chloroform-methanol-hexane-diethyl ether gave 50 mg (yield 42%) of Compound 9.

Example 10

5-Amino-2-(4-piperidino)phenyl-4H-benzopyran-4-one (Compound 10):

Sodium hydride (60% oil dispersion) (114 mg), 500 mg of 2-ethoxycarbonyl-N-pivaloyl-3-(2-tetrahydropyranyl)oxyaniline and 291 mg of 4-piperidinoacetophenone were treated in a similar manner as in Example 1 using toluene as the solvent. Silica gel column chromatography gave 515 mg of an oily product.

The oily product (527 mg) was cyclized in a similar manner as in Example 1 to give 276 mg (yield 55%) of 2-(4-piperidino)phenyl-5-(N-pivaloyl)amino-4H-benzopyran-4-one.

The obtained compound (204 mg) was then treated in a similar manner as in Example 2, followed by silica gel column chromatography. Subsequent recrystallization from chloroform-diethyl ether-hexane gave 129 mg (yield 79%) of Compound 10.

Example 11

5-Amino-2-(4-piperazino)phenyl-4H-benzopyran-4-one (Compound 11):

Sodium hydride (60% oil dispersion) (229 mg), 500 mg of 2-ethoxycarbonyl-N-pivaloyl-3-(2-tetrahydropyranyl)oxyaniline and 241 mg of 4-(4-acetyl)piperazinoacetophenone were treated in a similar manner as in Example 1 using toluene as the solvent. Silica gel column chromatography gave 312 mg of an oily product.

The oily product (308 mg) was cyclized in a similar manner as in Example 1 to give 158 mg (yield 63%) of 2-[4-(4-acetyl)piperazino]phenyl-5-(N-pivaloyl)amino-4H-benzopyran-4-one.

The obtained compound (158 mg) was then treated in a similar manner as in Example 2, followed by silica gel column chromatography. Subsequent recrystallization from chloroform-methanol gave 28 mg (yield 25%) of Compound 11.

Example 12

5-(N-Acetyl)amino-2-(4-dimethylamino)phenyl-4H-benzopyran-4-one (Compound 12):

In 2 ml of pyridine was dissolved 50 mg of Compound 8 obtained in Example 8, and 19 µl of acetyl chloride was added to the solution, followed by stirring at room temperature for 2 hours. Water was added to the reaction mixture and the precipitated insoluble matters were separated by filtration. Subsequent recrystallization from chloroform-ethyl acetate-hexane gave 51 mg (yield 87%) of Compound 12.

Example 13

2-[4-(N-Acetyl)amino]phenyl-5-amino-4H-benzopyran-4-one (Compound 13):

In 2 ml of pyridine was dissolved 100 mg of Compound 2 obtained in Example 2, and 40 µl of acetyl chloride was added to the solution, followed by stirring at 0°C for 20 minutes. The reaction mixture was dissolved in chloroform. After the solution was washed successively with a 10% aqueous solution of citric acid, a 5% aqueous solution of copper sulfate and a saturated aqueous solution of sodium chloride, the chloroform layer was concentrated under reduced pressure. The residue was subjected to silica gel column chromatography and then recrystallized from chloroform-methanol-hexane to give 44 mg (yield 37%) of Compound 13.

Example 14

5-Amino-2-(4-amino-2-hydroxy)phenyl-4H-benzopyran-4-one (Compound 14):

Sodium hydride (60% oil dispersion) (0.40 g), 1.9 g of 2-ethoxycarbonyl-N-pivaloyl-3-(2-tetrahydropyranyl)oxyaniline and 1.3 g of 4-(N-acetyl-N-methoxymethyl)amino-2-(methoxymethyl)-oxyacetophenone were treated in a similar manner as in Example 1. Silica gel column chromatography gave 0.58 g of an oily product.

The oily product (0.58 g) was cyclized in a similar manner as in Example 1 to give 0.18 g (yield 47%) of 2-[4-(N-acetyl)amino-2-hydroxy]phenyl-5-(N-pivaloyl)amino-4H-benzopyran-4-one.

The obtained compound (70 mg) was then treated in a similar manner as in Example 2, followed by silica gel column chromatography. Subsequent recrystallization from chloroform-hexane gave 29 mg (yield 61%) of Compound 14.

Example 15

5-Amino-2-(4-ethylamino)phenyl-4H-benzopyran-4-one (Compound 15):

Sodium hydride (60% oil dispersion) (0.78 g), 3.4 g of 2-ethoxycarbonyl-N-pivaloyl-3-(2-tetrahydropyranyl)oxyaniline and 2.0 g of 4-(N-acetyl-N-ethyl) aminoacetophenone were treated in a similar manner as in Example 1. Silica gel column chromatography gave 3.21 g of an oily product.

The oily product (3.11 g) was cyclized in a similar manner as in Example 1 to give 1.75 g (yield 68%) of 2-[4-(N-acetyl-N-ethyl)amino]phenyl-5-(N-pivaloyl)amino-4H-benzopyran-4-one.

The obtained compound (1.64 g) was then treated in a similar manner as in Example 2, followed by silica gel column chromatography. Subsequent recrystallization from chloroform-isopropyl ether gave 1.1 g (yield 72%) of Compound 15.

## Example 16

2-[4-(N-Acetyl-N-isopropyl)amino]phenyl-5-(N-pivaloyl)amino-4H-benzopyran-4-one (Compound 16):

Sodium hydride (60% oil dispersion) (0.64 g), 2.55 g of 2-ethoxycarbonyl-N-pivaloyl-3-(2-tetrahydropyranyl)oxyaniline and 1.28 g of 4-(N-acetyl-N-isopropyl)aminoacetophenone were treated in a similar manner as in Example 1. Silica gel column chromatography gave 2.26 g of an oily product.

The oily product (2.25 g) was cyclized in a similar manner as in Example 1, followed by recrystallization from ethyl acetate-hexane to give 0.59 g (yield 33%) of Compound 16.

## Example 17

2-[4-(N-Acetyl-N-isopropyl)amino]phenyl-5-amino-4H-benzopyran-4-one (Compound 17):

In 13 ml of ethanol was dissolved 1.59 g of Compound 16 obtained in Example 16, and 6.5 ml of concentrated hydrochloric acid was added to the solution. The mixture was stirred with heating under reflux for 2 hours. After the reaction mixture was concentrated under reduced pressure, the residue was dissolved in chloroform and the solution was washed successively with a saturated aqueous solution of sodium bicarbonate and water. The chloroform layer was dried over anhydrous sodium sulfate and concentrated under reduced pressure. The residue was subjected to silica gel column chromatography (eluent; chloroform : methanol = 30 : 1) and then recrystallized from ethyl acetate -diethyl ether to give 324 mg (yield 61%) of Compound 17.

## Example 18

5-Amino-2-[4-(isopropyl)amino]phenyl-4H-benzopyran-4-one (Compound 18):

In 3 ml of ethanol was dissolved 250 mg of Compound 17 obtained in Example 17, and 6 ml of a 47% aqueous solution of hydrobromic acid was added to the solution. The mixture was stirred with heating under reflux for 11.5 hours. After the reaction mixture was concentrated under reduced pressure, the residue was dissolved in chloroform and the solution was washed successively with a saturated aqueous solution of sodium bicarbonate and a saturated aqueous solution of sodium chloride. The chloroform layer was dried over anhydrous sodium sulfate and concentrated under reduced pressure. The residue was subjected to silica gel column chromatography (eluent; chloroform : methanol = 60 : 1) and then recrystallized from ethyl acetate-hexane to give 80 mg (yield 37%) of Compound 18.

## Example 19

5-Amino-2-[4-bis(2-hydroxyethyl)amino]phenyl-4H-benzopyran-4-one (Compound 19):

Sodium hydride (60% oil dispersion) (0.82 g), 3.92 g of 2-ethoxycarbonyl-N-pivaloyl-3-(2-tetrahydropyranyl)oxyaniline and 3.65 g of 4-bis(2-tetrahydropyranyloxyethyl)aminoacetopheone were treated in a similar manner as in Example 1. Silica gel column chromatography gave 5.77 g of an oily product.

The oily product (5.71 g) was cyclized in a similar manner as in Example 1 to give 2.25 g (yield 57%) of 2-[4-bis(2-hydroxyethyl)amino]phenyl-5-(N-pivaloyl)amino-4H-benzopyran-4-one.

The obtained compound (1.12 g) was then treated in a similar manner as in Example 2. The resulting reaction mixture was concentrated under reduced pressure and the residue was dissolved in chloroform. The solution was washed successively with a saturated aqueous solution of sodium bicarbonate and a saturated aqueous solution of sodium chloride, and dried over anhydrous sodium sulfate. After the chloroform layer was concentrated under reduced pressure, the residue was recrystallized from chloroform-methanol-hexane to give 0.66 g (yield 84%) of Compound 19.

## Example 20

2-[4-Bis(2-chloroethyl)amino]phenyl-5-(N-pivaloyl)amino-4H-benzopyran-4-one (Compound 20):

In 20 ml of chloroform was dissolved 1.0 g of 2-[4-bis(2-hydroxyethyl)amino]phenyl-5-(N-pivaloyl)amino-4H-benzopyran-4-one obtained in Example 19, and 10 ml of thionyl chloride was added to the solution. The mixture was stirred with heating under reflux for 2 hours. After the reaction mixture was concentrated under reduced pressure, the residue was dissolved in chloroform. The solution was washed successively with a saturated aqueous solution of sodium bicarbonate and a saturated aqueous solution of sodium chloride, followed by drying over anhydrous sodium sulfate. After the chloroform layer was concentrated under reduced pressure, the residue was subjected to silica gel column chromatography (eluent; hexane : ethyl acetate = 2.5 : 1) and then recrystallized from ethyl acetate-hexane to give 779 mg (yield 82%) of Compound 20.

## Example 21

5-Amino-2-[4-bis(2-chloroethyl)amino]phenyl-4H-benzopyran-4-one (Compound 21):

Compound 20 (0.64 g) obtained in Example 20 was treated in a similar manner as in Example 2 and then recrystallized from chloroform-methanol to give 105 mg (yield 18%) of the hydrochloride of Compound 21.

## Example 22

5-Amino-2-(4-propylamino)phenyl-4H-benzopyran-4-one (Compound 22):

Sodium hydride (60% oil dispersion) (1.38 g), 4.5 g of 2-ethoxycarbonyl-N-pivaloyl-3-(2-tetrahydropyranyl)oxyaniline and 1.88 g of 4-(N-acetyl-N-propyl)aminoacetophenone were treated in a similar manner as in Example 1. Silica gel column chromatography gave 2.64 g of an oily product.

The oily product (2.64 g) was cyclized in a similar manner as in Example 1 to give crude 2-[4-(N-acetyl-N-propyl)amino]phenyl-5-(N-pivaloyl)amino-4H-benzopyran-4-one. Without further purification, the obtained compound was treated in a similar manner as in Example 2, followed by silica gel column chromatography. Then, the compound was converted into the hydrochloride with hydrogen chloride in ethyl acetate-isopropanol. Subsequent recrystallization from chloroform-methanol-diethyl ether gave 0.43 g (yield 29%) of the hydrochloride of Compound 22.

## Example 23

5-Amino-2-(4-butylamino)phenyl-4H-benzopyran-4-one (Compound 23):

Sodium hydride (60% oil dispersion) (1.38 g), 3.0 g of 2-ethoxycarbonyl-N-pivaloyl-3-(2-tetrahydropyranyl)oxyaniline and 2.0 g of 4-(N-acetyl-N-butyl)aminoacetophenone were treated in a similar manner as in Example 1. Silica gel column chromatography gave 2.75 g of an oily product.

The oily product (2.75 g) was cyclized in a similar manner as in Example 1 to give 2.18 g (yield 95%) of 2-[4-(N-acetyl-N-butyl)amino]phenyl-5-(N-pivaloyl)amino-4H-benzopyran-4-one.

The obtained compound (2.18 g) was treated in a similar manner as in Example 2, followed by silica gel column chromatography. Subsequent recrystallization from ethyl acetate gave 0.68 g (yield 44%) of Compound 23.

Example 24

5-Amino-2-(4-benzylamino)phenyl-4H-benzopyran-4-one (Compound 24):

In 50 ml of dimethylformamide was dissolved 568 mg of Compound 6 obtained in Example 6, and 379 mg of potassium tert-butoxide and 501 µl of benzyl bromide were added to the solution. The mixture was stirred at room temperature for 6.5 hours. After the reaction mixture was concentrated under reduced pressure, the residue was dissolved in chloroform. The solution was washed successively with water and a saturated aqueous solution of sodium chloride, and then dried over anhydrous sodium sulfate. The chloroform layer was concentrated under reduced pressure and the residue was subjected to silica gel column chromatography (eluent; chloroform : methanol = 20 : 1) to give 225 mg (yield 31%) of 5-acetylamino-2-[4-(N-acetyl-N-benzyl)phenyl]-4H-benzopyran-4-one.

The obtained compound (210 mg) was treated in a similar manner as in Example 2. Subsequent recrystallization from chloroform-methanol gave 173 mg (yield 85%) of the hydrochloride of Compound 24.

Example 25

5-Amino-2-(4-carboxymethylamino)phenyl-4H-benzopyran-4-one (Compound 25):

Compound 41 (700 mg) obtained in Example 41 and 447 µl of tert-butyl bromoacetate were treated in a similar manner as in Example 24 to give 681 mg (yield 77%) of 2-[4-(N-acetyl-N-tert-butoxycarbonylmethyl)amino]phenyl-5-(N-pivaloyl)amino-4H-benzopyran-4-one.

The obtained compound (470 mg) was treated in a similar manner as in Example 2. The product was subjected to silica gel column chromatography and then slurried with ethyl acetate to give 55 mg (yield 18%) of Compound 25.

Example 26

5-Amino-2-[4-(N-ethoxycarbonylmethyl)amino]phenyl-4H-benzopyran-4-one (Compound 26):

In 50 ml of ethanol was dissolved 500 mg of Compound 25 obtained in Example 25, and 5 ml of concentrated hydrochloric acid was added to the solution, followed by stirring at 60 °C for 2 hours. After the reaction mixture was concentrated under reduced pressure, the residue was subjected to silica gel column chromatography and then recrystallized from hexane-ethyl acetate to give 485 mg (yield 89%) of Compound 26.

Example 27

5-Amino-2-[4-(N-methoxycarbonylmethyl)amino]phenyl-4H-benzopyran-4-one (Compound 27):

Compound 25 (500 mg) obtained in Example 25 was dissolved in 50 ml of methanol and then treated in

a similar manner as in Example 26 to give 449 mg (yield 85%) of Compound 27.

Example 28

5-Amino-2-[4-(2-hydroxyethyl)amino]phenyl-4H-benzopyran-4-one (Compound 28):

Sodium hydride (60% oil dispersion) (3.43 g), 15 g of 2-ethoxycarbonyl-N-pivaloyl-3-(2-tetrahydropyranyl)oxyaniline and 10.5 g of 4-[N-acetyl-N-(2-tetrahydropyranyloxy)ethyl]aminoacetophenone were treated in a similar manner as in Example 1. Silica gel column chromatography gave 4.64 g of an oily product.

The oily product (4.64 g) was cyclized in a similar manner as in Example 1 to give 5.20 g (yield 68%) of 2-[4-(2-hydroxyethyl)amino]phenyl-5-(N-pivaloyl)amino-4H-benzopyran-4-one.

The obtained compound (1.0 g) was treated in a similar manner as in Example 2, followed by silica gel column chromatography. Subsequent recrystallization from chloroform-methanol gave 0.61 g (yield 78%) of Compound 28.

Example 29

5-Amino-2-(4-amino-3-hydroxy)phenyl-4H-benzopyran-4-one (Compound 29):

Sodium hydride (60% oil dispersion) (175 mg), 764 mg of 2-ethoxycarbonyl-N-pivaloyl-3-(2-tetrahydropyranyl)oxyaniline and 488 mg of 3-(methoxymethyl)oxy-4-(N-pivaloyl)aminoacetophenone were treated in a similar manner as in Example 1. Silica gel column chromatography gave 545 mg of an oily product.

The oily product (545 mg) was cyclized in a similar manner as in Example 1 to give 197 mg (yield 44%) of 2-[3-(methoxymethyl)oxy-4-(N-pivaloyl)amino]phenyl-5-(N-pivaloyl)amino-4H-benzopyran-4-one and 215 mg (yield 52%) of 2-[3-hydroxy-4-(N-pivaloyl)amino]phenyl-5-(N-pivaloyl)amino-4H-benzopyran-4-one.

The obtained compounds (333 mg in total) were treated in a similar manner as in Example 2, followed by silica gel column chromatography. Subsequent recrystallization from chloroform-methanol gave 70 mg (yield 36%) of Compound 29.

Example 30

2-[3-Fluoro-4-(N-pivaloyl)amino]phenyl-5-(N-pivaloyl)amino-4H-benzopyran-4-one (Compound 30):

Sodium hydride (60% oil dispersion) (366 mg), 3.20 g of 2-ethoxycarbonyl-N-pivaloyl-3-(2-tetrahydropyranyl)oxyaniline and 1.09 g of 3-fluoro-4-(N-pivaloyl)aminoacetophenone were treated in a similar manner as in Example 1 using toluene as the solvent. Silica gel column chromatography gave 0.53 g of an oily product.

The oily product (0.52 g) was cyclized in a similar manner as in Example 1. Subsequent recrystallization from ethyl acetate gave 249 mg (yield 59%) of Compound 30.

Example 31

5-Amino-2-(4-amino-3-fluoro)phenyl-4H-benzopyran-4-one (Compound 31):

Compound 30 (276 mg) obtained in Example 30 was treated in a similar manner as in Example 2 and then recrystallized from methanol to give 170 mg (yield 79%) of the hydrochloride of Compound 31.

Example 32

2-[3,5-Difluoro-4-(N-pivaloyl)amino]phenyl-5-(N-pivaloyl)amino-4H-benzopyran-4-one (Compound 32):

Sodium hydride (60% oil dispersion) (1.23 g), 5.39 g of 2-ethoxycarbonyl-N-pivaloyl-3-(2-tetrahydropyranyl)oxyaniline and 1.97 g of 3,5-difluoro-4-(N-pivaloyl)aminoacetophenone were treated in a similar manner as in Example 1 using a solvent mixture of toluene and dioxane. Silica gel column chromatography gave 1.95 g of an oily product.

The oily product (1.95 g) was cyclized in a similar manner as in Example 1. Subsequent recrystallization from ethyl acetate gave 1.06 g (yield 77%) of Compound 32.

Example 33

5-Amino-2-(4-amino-3,5-difluoro)phenyl-4H-benzopyran-4-one (Compound 33):

In 5 ml of dioxane was dissolved 0.87 g of Compound 32 obtained in Example 32, and 5 ml of concentrated hydrochloric acid was added to the solution. The mixture was stirred with heating under reflux for 7 hours. After the reaction mixture was concentrated under reduced pressure, the residue was recrystallized from methanol to give 273 mg (yield 36%) of the hydrochloride of Compound 33.

Example 34

2-[4-(2-Methoxyethyl)amino]phenyl-5-(N-pivaloyl)amino-4H-benzopyran-4-one (Compound 34):

In 10 ml of tetrahydrofuran was dissolved 500 mg of 2-[4-(2-hydroxyethyl)amino]phenyl-5-(N-pivaloyl)-amino-4H-benzopyran-4-one obtained in Example 28, and 58 mg of sodium hydride (60% oil dispersion) and 2 ml of methyl iodide were added to the solution. The mixture was stirred at 50 °C for one hour. After the reaction solution was concentrated under reduced pressure, the residue was dissolved in chloroform. The solution was washed successively with water and a saturated aqueous solution of sodium chloride, and then dried over anhydrous sodium sulfate. The chloroform layer was concentrated under reduced pressure and the residue was purified by silica gel column chromatography (eluent; hexane : ethyl acetate = 3 : 2) to give 496 mg (yield 96%) of Compound 34.

Example 35

5-Amino-2-[4-(2-methoxyethyl)amino]phenyl-4H-benzopyran-4-one (Compound 35):

Compound 34 (260 mg) obtained in Example 34 was treated in a similar manner as in Example 2, followed by silica gel column chromatography. Subsequent recrystallization from hexane-ethyl acetate gave 184 mg (yield 90%) of Compound 35.

Example 36

5-Amino-2-(4-amino)phenyl-8-methoxy-4H-benzopyran-4-one (Compound 36):

Sodium hydride (60% oil dispersion) (1.23 g), 5.56 g of 2-ethoxycarbonyl-N-pivaloyl-4-methoxy-3-(2-tetrahydropyranyl)oxyaniline and 3.21 g of 4-(N-pivaloyl)aminoacetophenone were treated in a similar manner as in Example 1. Silica gel column chromatography gave 6.86 g of an oily product.

The oily product (6.86 g) was cyclized in a similar manner as in Example 1 to give 3.60 g (yield 56%)

of 5-(N-pivaloyl)amino-2-[4-(N-pivaloyl)amino]phenyl-8-methoxy-4H-benzopyran-4-one.

The obtained compound (1.50 g) was treated in a similar manner as in Example 2. Subsequent neutralization and recrystallization from ethanol gave 0.53 g (yield 56%) of Compound 36.

Example 37

5-Amino-2-(4-methylamino)phenyl-8-methoxy-4H-benzopyran-4-one (Compound 37):

Sodium hydride (60% oil dispersion) (0.21 g), 1.0 g of 2-ethoxycarbonyl-N-pivaloyl-4-methoxy-3-(2-tetrahydropyranyl)oxyaniline and 0.50 g of 4-(N-acetyl-N-methyl)aminoacetophenone were treated in a similar manner as in Example 1. Silica gel column chromatography gave 0.70 g of an oily product.

The oily product (231 mg) was cyclized in a similar manner as in Example 1 to give 135 mg (yield 12%) of 2-[4-(N-acetyl-N-methyl)amino]phenyl-5-(N-pivaloyl)amino-8-methoxy-4H-benzopyran-4-one.

The obtained compound (125 mg) was treated in a similar manner as in Example 2 to give 39 mg (yield 56%) of the hydrochloride of Compound 37.

Example 38

5-Amino-2-[4-(2-bromoethyl)amino]phenyl-4H-benzopyran-4-one (Compound 38):

In 20 ml of tetrahydrofuran was dissolved 665 mg of Compound 42 obtained in Example 42, and 802 mg of tetrabutylammonium bromide was added to the solution. The mixture was stirred with heating under reflux for 30 minutes, followed by addition of ethyl acetate. The reaction mixture was washed with a saturated aqueous solution of sodium chloride, dried over anhydrous sodium sulfate, and then concentrated under reduced pressure. The residue was subjected to silica gel column chromatography (eluent; hexane : ethyl acetate = 3 : 2) to give 476 mg (yield 78%) of 2-[4-(2-bromoethyl)amino]phenyl-5-(N-pivaloyl)amino-4H-benzopyran-4-one.

The obtained compound (326 mg) was dissolved in 3 ml of ethanol, and 3 ml of a 48% aqueous solution of hydrobromic acid was added to the solution. The mixture was stirred with heating under reflux for 3 hours. After the reaction mixture was concentrated under reduced pressure, the residue was dissolved in chloroform. The solution was washed successively with a saturated aqueous solution of sodium bicarbonate and water, and dried over anhydrous sodium sulfate. The chloroform layer was concentrated under reduced pressure, and the residue was purified by silica gel column chromatography and then recrystallized from methanol-diethyl ether to give 129 mg (yield 49%) of Compound 38.

Example 39

5-Amino-2-{4-[N-(2-chloroethyl)]amino}phenyl-4H-benzopyran-4-one (Compound 39):

In 20 ml of tetrahydrofuran was dissolved 1 g of Compound 42 obtained in Example 42, and 299 mg of sodium hydride (60% oil dispersion) was added to the solution. The mixture was stirred with heating under reflux for 2 hours, followed by addition of 50 ml of ethyl acetate. The reaction mixture was washed successively with water and a saturated aqueous solution of sodium chloride, dried over anhydrous sodium sulfate, and then concentrated under reduced pressure. The residue was subjected to silica gel column chromatography (eluent; hexane : ethyl acetate = 1 : 1) to give 304 mg (yield 42%) of 2-(4-aziridino)-phenyl-5-(N-pivaloyl)amino-4H-benzopyran-4-one.

The obtained compound (294 mg) was treated in a similar manner as in Example 2, followed by silica gel column chromatography. Subsequent recrystallization from ethyl acetate -hexane gave 149 mg (yiled 57%) of Compound 39.

Example 40

5-Amino-2-(4-aziridino)phenyl-4H-benzopyran-4-one (Compound 40):

In 3 ml of dimethylformamide was dissolved 93 mg of Compound 38 obtained in Example 38, and 42 mg of sodium hydride (60% oil dispersion) was added to the solution, followed by stirring at 60°C for 3 hours. After the reaction mixture was concentrated under reduced pressure, the residue was dissolved in dichloromethane. The solution was washed successively with water and a saturated aqueous solution of sodium chloride, dried over anhydrous sodium sulfate, and then concentrated under reduced pressure. The residue was purified by silica gel column chromatography (eluent; hexane : ethyl acetate = 1 : 1) to give 46 mg (yield 64%) of Compound 40.

Example 41

2-[4-(N-Acetyl)amino]phenyl-5-(N-pivaloyl)amino-4H-benzopyran-4-one (Compound 41):

Sodium hydride (60% oil dispersion) (3.71 g), 16.2 g of 2-ethoxycarbonyl-N-pivaloyl-3-(2-tetrahydropyranyl)oxyaniline and 10.3 g of 4-(N-acetyl-N-methoxymethyl)aminoacetophenone were treated in a similar manner as in Example 1. Silica gel column chromatography gave 7.08 g of an oily product.

The oily product (7.08 g) was cyclized in a similar manner as in Example 1. Subsequent recrystallization from chloroform-methanol gave 2.68 g (yield 52%) of Compound 41.

Example 42

2-{4-[2-(4-Methylphenylsulfonyloxy)ethyl]amino}phenyl-5-(N-pivaloyl)amino-4H-benzopyran-4-one (Compound 42):

In 100 ml of tetrahydrofuran was dissolved 2.93 g of 2-[4-(2-hydroxyethyl)amino]phenyl-5-(N-pivaloyl)-amino-4H-benzopyran-4-one obtained in Example 28, and 578 mg of sodium hydride (60% oil dispersion) and 3.0 g of p-toluenesulfonyl chloride were added to the solution. The mixture was stirred at room temperature for 18 hours. After the reaction mixture was concentrated under reduced pressure, the residue was dissolved in chloroform. The solution was washed successively with a saturated aqueous solution of sodium bicarbonate and a saturated aqueous solution of sodium chloride, and then dried over anhydrous sodium sulfate. The chloroform layer was concentrated under reduced pressure and the residue was purified by silica gel column chromatography to give 2.42 g (yield 59%) of Compound 42.

Example 43

5-Amino-2-{4-[N-benzyl-N-(2-benzyloxyethyl)]amino}phenyl-4H-benzopyran-4-one (Compound 43):

In 10 ml of tetrahydrofuran was dissolved 500 mg of 2-[4-(2-hydroxyethyl)amino]phenyl-5-(N-pivaloyl)-amino-4H-benzopyran-4-one obtained in Example 28, and 79 mg of sodium hydride (60% oil dispersion) and 470 μl of benzyl bromide were added to the solution. The mixture was stirred at 70 to 80°C for 5 hours, followed by addition of chloroform. The resulting solution was washed successively with a 10% aqueous solution of citric acid and a saturated aqueous solution of sodium chloride, dried over anhydrous sodium sulfate, and then concentrated under reduced pressure. The residue was subjected to silica gel column chromatography (eluent; hexane : ethyl acetate = 3 : 1) to give 341 mg (yield 64%) of 2-{4-[N-benzyl-N-(2-benzyloxyethyl)]amino}phenyl-5-(N-pivaloyl)amino-4H-benzopyran-4-one (Compound e) and 71 mg (yield 13%) of 2-[4-(2-benzyloxyethyl)amino]phenyl-5-(N-pivaloyl)amino-4H-benzopyran-4-one (Compound f).

Compound e (340 mg) was treated in a similar manner as in Example 2, followed by silica gel column chromatography. Subsequent recrystallization from ethyl acetate-diethyl ether gave 84 mg (yield 30%) of Compound 43.

Example 44

5-Amino-2-[4-(2-benzyloxyethyl)amino]phenyl-4H-benzopyran-4-one (Compound 44):

Compound f (71 mg) obtained in Example 43 was treated in a similar manner as in Example 2. The product was subjected to silica gel column chromatography and then slurried with diethyl ether to give 8 mg (yield 14%) of Compound 44.

Example 45

5-Amino-2-[4-(2-ethoxyethyl)amino]phenyl-4H-benzopyran-4-one (Compound 45):

In 10 ml of tetrahydrofuran was dissolved 500 mg of 2-[4-(2-hydroxyethyl)amino]phenyl-5-(N-pivaloyl)-amino-4H-benzopyran-4-one obtained in Example 28, and 57 mg of sodium hydride (60% oil dispersion) and 2 ml of ethyl iodide were added to the solution. The mixture was stirred at room temperature for 5 hours, followed by addition of chloroform. The resulting solution was washed successively with a 10% aqueous solution of citric acid and a saturated aqueous solution of sodium chloride, dried over anhydrous sodium sulfate, and then concentrated under reduced pressure. The residue was subjected to silica gel column chromatography (eluent; hexane : ethyl acetate = 2 : 1) to give 246 mg (yield 46%) of 2-[4-(2-ethoxyethyl)amino]phenyl-5-(N-pivaloyl)amino-4H-benzopyran-4-one.

The obtained compound (232 mg) was treated in a similar manner as in Example 2, followed by silica gel column chromatography. Subsequent recrystallization from ethyl acetate-diethyl ether gave 130 mg (yield 71%) of Compound 45.

Example 46

5-(N-Acetyl)amino-2-{4-[N-acetyl-N-(2-methoxyethyl)]amino}phenyl-4H-benzopyran-4-one (Compound 46):

In 1 ml of pyridine was dissolved 139 mg of Compound 35 obtained in Example 35, and 0.5 ml of acetic anhydride and 47 µl of acetyl chloride were added to the solution. The mixture was stirred at room temperature for 2 hours, followed by addition of chloroform. The resulting solution was washed successively with a 10% aqueous solution of citric acid and a saturated aqueous solution of sodium chloride, dried over anhydrous sodium sulfate, and then concentrated under reduced pressure. The residue was subjected to silica gel column chromatography and then recrystallized from ethyl acetate -diethyl ether to give 120 mg (yield 68%) of Compound 46.

Example 47

5-Amino-2-(4-amino)phenyl-8-hydroxy-4H-benzopyran-4-one (Compound 47):

In 25 ml of dichloromethane was dissolved 286 mg of Compound 36 obtained in Example 36, and 5.6 ml of boron tribromide (1M dichloromethane solution) was added to the solution at -70 °C in an argon atmosphere, followed by stirring for 1.5 hours. The mixture was stirred at room temperature for further 9 hours. The reaction mixture was then poured into ice water and dichloromethane was added thereto. To the aqueous layer was added a 2N aqueous solution of sodium hydroxide to adjust pH to 9.0. The formed precipitates were separated by filtration and then recrystallized from ethyl alcohol-hexane to give 166 mg (yield 61%) of Compound 47.

Example 48

2-(4-Hydroxy)phenyl-5-(N-pivaloyl)amino-4H-benzopyran-4-one (Compound 48):

In 10 ml of dioxane was suspended 0.46 g of sodium hydride (60% oil dispersion). A solution of 2.0 g of 2-ethoxycarbonyl-N-pivaloyl-3-(2-tetrahydropyranyl)oxyaniline and 1.26 g of Compound a obtained in Reference Example 1 in 10 ml of dioxane was added dropwise to the suspension with heating under reflux. The mixture was heated under reflux for further 3.5 hours. After the reaction mixture was cooled, water was added thereto, followed by extraction with ethyl acetate. The ethyl acetate layer was washed with water, dried over anhydrous sodium sulfate, and concentrated to give 3.3 g of an oily substance.

The oily substance was dissolved in 30 ml of ethanol without purification and 1.9 ml of concentrated hydrochloric acid was added to the solution, followed by stirring at room temperature for 50 minutes. After addition of an aqueous solution of sodium chloride, the reaction mixture was extracted with chloroform. The chloroform layer was washed successively with a 5% aqueous solution of sodium bicarbonate and water, dried over anhydrous sodium sulfate, and then concentrated to give 1.80 g of an oily substance.

The oily substance was dissolved in 30 ml of isopropanol without purification and 1.8 g of Amberlist 15 was added to the solution, followed by heating under reflux for one hour. After Amberlist 15 was removed by filtration, the reaction mixture was concentrated. The residue was purified by silica gel column chromatography (eluent; chloroform : acetone = 20 : 1) to give 0.56 g (yield based on the starting compound 29%) of Compound 48.

Example 49

5-Amino-2-(4-hydroxy)phenyl-4H-benzopyran-4-one (Compound 49):

Compound 48 (0.33 g) obtained in Example 48 was heated under reflux in 10 ml of ethanol and 5 ml of concentrated hydrochloric acid for 6 hours. The reaction mixture was cooled, and the precipitated crystals were separated by filtration and washed with cold ethanol to give 0.23 g (yield 77%) of the hydrochloride of Compound 49.

Example 50

5-Amino-2-(3-hydroxy)phenyl-4H- benzopyran-4-one (Compound 50):

Compound 50 was obtained in a similar manner as in Examples 48 and 49.

Example 51

5-Amino-2-(3,5-dihydroxy)phenyl-4H-benzopyran-4-one (Compound 51):

Compound 51 was obtained in a similar manner as in Examples 48 and 49.

Example 52

5-Amino-2-[4-(2-dimethylaminoethyl)oxy]phenyl-4H-benzopyran-4-one (Compound 52):

In 3 ml of dimethylformamide were suspended 100 mg of Compound 49 obtained in Example 49 and 114 mg of N,N-dimethylaminoethyl chloride hydrochloride, and 55 mg of sodium hydride (60% oil dispersion) was added to the suspension, followed by stirring at 50 °C. After three hours, 10 mg of sodium hydride and 15 mg of sodium iodide were added to the reaction mixture, followed by stirring at 50 °C for further 2.5 hours. After addition of an aqueous solution of sodium chloride, the reaction mixture was extracted with chloroform. The chloroform layer was washed with water, dried over anhydrous sodium

sulfate, and then concentrated. The residue was purified by preparative silica gel thin layer chromatography (developing solvent; chloroform : methanol = 4 : 1) to give 49 mg (yield 38%) of Compound 52. The compound was converted into the hydrochloride in a conventional manner.

## Example 53

5-Amino-2-[3-(2-dimethylaminoethyl)oxy]phenyl-4H-benzopyran-4-one (Compound 53):

Compound 53 (78 mg, yield 41%) was obtained from 150 mg of Compound 50 obtained in Example 50 in a similar manner as in Example 52.

## Example 54

8-Methoxy-2-(3,4-dimethoxy)phenyl-5-(N-pivaloyl)amino-4H-benzopyran-4-one (Compound 54):

In 5 ml of dioxane was suspended 0.42 g of sodium hydride (60% oil dispersion). A solution of 2 g of 2-ethoxycarbonyl-4-methoxy-N-pivaloyl-3-(2-tetrahydropyranyl)oxyaniline and 0.96 g of 3,4-dimethoxyacetophenone in 6 ml of dioxane was added dropwise to the suspension with heating under reflux. The mixture was heated under reflux for further 4 hours. Water was added to the reaction mixture and an oily substance was extracted with petroleum ether. The aqueous layer was neutralized with diluted hydrochloric acid, and extracted with ethyl acetate. Then the ethyl acetate layer was washed with water, dried over anhydrous sodium sulfate, and concentrated to give 1.93 g of an oily substance.

The oily substance was dissolved in 20 ml of ethanol without purification, and 1.2 ml of concentrated hydrochloric acid was added to the solution, followed by stirring at room temperature for 40 minutes. The reaction mixture was cooled, and the precipitated crystals were separated by filtration and washed with cold ethanol to give 0.66 g (yield 43%) of Compound 54.

## Example 55

5-Amino-8-methoxy-2-(3,4-dimethoxy)phenyl-4H-benzopyran-4-one (Compound 55):

To 0.66 g of Compound 54 obtained in Example 54 were added 20 ml of dioxane and 10 ml of concentrated hydrochloric acid, followed by heating under reflux for 7 hours. The reaction mixture was cooled, and the precipitated crystals were separated by filtration and washed with cold ethanol to give 0.49 g (yield 83%) of the hydrochloride of Compound 55.

## Example 56

5-Amino-8-methoxy-2-phenyl-4H-benzopyran-4-one (Compound 56):

Compound 56 was obtained in a similar manner as in Examples 54 and 55.

## Example 57

5-Amino-8-methoxy-2-(3-methoxy)phenyl-4H-benzopyran-4-one (Compound 57):

Compound 57 was obtained in a similar manner as in Examples 54 and 55.

Example 58

5-Amino-8-methoxy-2-(4-methoxy)phenyl-4H-benzopyran-4-one (Compound 58):

Compound 58 was obtained in a similar manner as in Examples 54 and 55.

Example 59

5-Amino-2-(3-methoxy)phenyl-4H-benzopyran-4-one (Compound 59):

In 30 ml of isopropanol was dissolved 2.51 g of Compound b obtained in Reference Example 2, and 2.5 g of Amberlist 15 was added to the solution, followed by heating under reflux for 1.5 hours. After Amberlist 15 was removed by filtration, the reaction mixture was concentrated to give 2.47 g of an amorphous solid.

To 0.93 g of the amorphous solid were added 20 ml of ethanol and 10 ml of concentrated hydrochloric acid. The mixture was heated under reflux for 7 hours. The reaction mixture was cooled and the precipitated solid was separated by filtration. The solid was washed with cold ethanol and then recrystallized from ethanol-chloroform to give 0.41 g (yield 56%) of the hydrochloride of Compound 59.

Example 60

5-Amino-2-phenyl-4H-benzopyran-4-one (Compound 60):

Compound 60 was obtained in a similar manner as in Example 59.

Example 61

5-Hydrazino-2-phenyl-4H-benzopyran-4-one (Compound 61):

In 20 ml of acetic acid was dissolved 1.00 g of Compound 60 obtained in Example 60. To the solution was added dropwise 3 ml of an aqueous solution of 0.35 g of sodium nitrite under cooling at about -10°C, followed by stirring at the same temperature for 15 minutes. Then, a solution of 2.37 g of stannous chloride dihydrate in 5 ml of concentrated hydrochloric acid was added dropwise thereto, followed by stirring at room temperature for 1.5 hours. The reaction mixture was poured into 150 ml of ice water and the formed solid was separated by filtration. The solid was suspended in 100 ml of a saturated aqueous solution of sodium bicarbonate and the suspension was extracted with 200 ml of ethyl acetate. The ethyl acetate layer was washed with water, dried over anhydrous sodium sulfate, and then concentrated. The residue was purified by silica gel column chromatography (eluent; chloroform : acetone = 40 : 1) to give 0.44 g (yield 42%) of Compound 61. The compound was converted into the hydrochloride in a conventional manner.

Example 62

5-(2-Dimethylaminoethyl)amino-2-phenyl-4H-benzopyran-4-one (Compound 62):

In 15 ml of tetrahydrofuran were suspended 326 mg of Compound 60 obtained in Example 60 and 792 mg of N,N-dimethylaminoethyl chloride hydrochloride, and 330 mg of sodium hydride (60% oil dispersion) was added to the suspension at 0°C. After addition of 30 mg of sodium iodide, the reaction mixture was stirred at 60°C for 7 hours. The reaction mixture was then poured into an aqueous solution of ammonium chloride and the mixture was extracted with chloroform. The chloroform layer was washed with water, dried over anhydrous sodium sulfate, and then concentrated. The residue was purified by silica gel column

chromatography (eluent; chloroform : methanol = 9 : 1) to give 63 mg (yield 15%) of Compound 62.

The compound was converted into the hydrochloride in a conventional manner.

Example 63

5-(N′-Acetyl)hydrazino-2-phenyl-4H-benzopyran-4-one (Compound 63):

In 1.5 ml of acetic acid was dissolved 56 mg of Compound 61 obtained in Example 61, and the solution was stirred at 65°C for 2 hours. The reaction mixture was poured into water, followed by extraction with chloroform. The chloroform layer was washed successively with water and a saturated aqueous solution of sodium bicarbonate, dried over anhydrous sodium sulfate, and then concentrated. The obtained solid was purified by preparative silica gel thin layer chromatography (developing solvent; chloroform : methanol = 9 : 1) to give 45 mg (yield 69%) of Compound 63.

Example 64

5-Amino-2-(3,4-dimethoxy)phenyl-4H-benzopyran-4-one (Compound 64):

Sodium hydride (60% oil dispersion) (0.46 g), 2.0 g of 2-ethoxycarbonyl-N-pivaloyl-3-(2-tetrahydropyranyl)oxyaniline and 1.03 g of 3,4-dimethoxyacetophenone were treated in a similar manner as in Reference Example 2 to give 2.05 g (yield 74%) of 1-(3,4-dimethoxy)phenyl-3-[2-(2-tetrahydropyranyl)-oxy-6-(N-pivaloyl)amino]phenylpropan-1,3-dione.

The obtained compound was dissolved in 20 ml of ethanol and 1.2 ml of concentrated hydrochloric acid was added to the solution, followed by stirring at room temperature for 30 minutes. The formed crystals were separated by filtration and washed with ethanol to give 499 mg (yield 31%) of 2-(3,4-dimethoxy)-phenyl-5-(N-pivaloyl)amino-4H-benzopyran-4-one.

The compound thus obtained was dissolved in 60 ml of ethanol and 20 ml of concentrated hydrochloric acid was added to the solution, followed by stirring with heating under reflux for 6 hours. The reaction mixture was cooled and the formed crystals were recrystallized from ethanol to give 174 mg (yield 40%) of the hydrochloride of Compound 64.

Example 65

5-Amino-2-(3,5-dimethoxy-4-hydroxy)phenyl-4H-benzopyran-4-one (Compound 65):

Sodium hydride (60% oil dispersion) (0.50 g), 2.18 g of 2-ethoxycarbonyl-N-pivaloyl-3-(2-tetrahydropyranyl)oxyaniline and 1.75 g of Compound c obtained in Reference Example 3 were treated in a similar manner as in Reference Example 2 to give 2.38 g (yield 65%) of 1-[3,5-dimethoxy-4-(2-tetrahydropyranyl)oxy]phenyl-3-[2-(2-tetrahydropyranyl)oxy-6-(N-pivaloyl)amino]phenylpropan-1,3-dione.

The obtained compound was treated in a similar manner as in Example 64 to give 1.11 g (yield 68%) of 2-(3,5-dimethoxy-4-hydroxy)phenyl-5-(N-pivaloyl)amino-4H-benzopyran-4-one.

The compound thus obtained was treated in a similar manner as in Example 64 to give 371 mg (yield 38%) of the hydrochloride of Compound 65.

Example 66

5-Amino-2-(2,4-dimethoxy)phenyl-4H-benzopyran-4-one (Compound 66):

Sodium hydride (60% oil dispersion) (0.92 g), 4.0 g of 2-ethoxycarbonyl-N-pivaloyl-3-(2-tetrahydropyranyl)oxyaniline and 2.06 g of 2,4-dimethoxyacetophenone were treated in a similar manner as

in Reference Example 2 to give 4.04 g (yield 73%) of 1-(2,4-dimethoxy)phenyl-3-[2-(2-tetrahydropyranyl)-oxy-6-(N-pivaloyl)amino]phenylpropan-1,3-dione.

The obtained compound was treated in a similar manner as in Example 64 to give 664 mg (yield 21%) of 2-(2,4-dimethoxy)phenyl-5-(N-pivaloyl)amino-4H-benzopyran-4-one.

The compound thus obtained was treated in a similar manner as in Example 64 to give 482 mg (yield 83%) of the hydrochloride of Compound 66.

Example 67

5-Amino-2-(3,5-dimethoxy)phenyl-4H-benzopyran-4-one (Compound 67):

Sodium hydride (60% oil dispersion) (0.46 g), 2.0 g of 2-ethoxycarbonyl-N-pivaloyl-3-(2-tetrahydropyranyl)oxyaniline and 1.03 g of 3,5-dimethoxyacetophenone were treated in a similar manner as in Reference Example 2 to give 2.01 g (yield 73%) of 1-(3,5-dimethoxy)phenyl-3-[2-(2-tetrahydropyranyl)-oxy-6-(N-pivaloyl)amino]phenylpropan-1,3-dione.

The obtained compound was dissolved in 15 ml of ethanol and 1 ml of concentrated hydrochloric acid was added to the solution, followed by stirring at room temperature for one hour. The reaction mixture was concentrated under reduced pressure and the residue was purified by silica gel column chromatography (eluent; hexane : ethyl acetate = 4 : 1) to give 0.47 g (yield 30%) of 2-(3,5- dimethoxy)phenyl-5-(N-pivaloyl)amino-4H-benzopyran-4-one.

The compound thus obtained was treated in a similar manner as in Example 64 to give 333 mg (yield 81%) of the hydrochloride of Compound 67.

Example 68

5-Amino-2-(2,4-dihydroxy)phenyl-4H-benzopyran-4-one (Compound 68):

Sodium hydride (60% oil dispersion) (0.46 g), 2.0 g of 2-ethoxycarbonyl-N-pivaloyl-3-(2-tetrahydropyranyl)oxyaniline and 1.38 g of Compound d obtained in Reference Example 4 were treated in a similar manner as in Reference Example 2 to give 2.32 g (yield 75%) of 1-(2,4-dimethoxymethyloxy)phenyl-3-[2-(2-tetrahydropyranyl)oxy-6-(N-pivaloyl)amino]phenylpropan-1,3-dione.

The obtained compound (0.92 g) was dissolved in 10 ml of tetrahydrofuran and 2 ml of concentrated hydrochloric acid was added to the solution, followed by stirring at room temperature for 3 hours. The reaction mixture was concentrated under reduced pressure and the residue was purified by silica gel column chromatography (eluent; chloroform :methanol = 15 : 1) to give 0.22 g (yield 36%) of 2-(2,4-dihydroxy)phenyl-5-(N-pivaloyl)amino-4H-benzopyran-4-one.

The compound thus obtained was dissolved in 45 ml of ethanol and 20 ml of concentrated hydrochloric acid was added to the solution, followed by stirring with heating under reflux for 4 hours. The reaction mixture was cooled and the formed crystals were purified by silica gel column chromatography (eluent; chloroform : methanol : water = 80 : 30 : 3) to give 145 mg (yield 87%) of Compound 68.

Example 69

5-Amino-2-(4-methoxy)phenyl-4H-benzopyran-4-one (Compound 69):

Sodium hydride (60% oil dispersion) (0.27 g), 1.2 g of 2-ethoxycarbonyl-N-pivaloyl-3-(2-tetrahydropyranyl)oxyaniline and 0.57 g of 4-methoxyacetophenone were treated in a similar manner as in Reference Example 2 to give 0.50 g (yield 32%) of 1-(4-methoxy)phenyl-3-[2-(2-tetrahydropyranyl)oxy-6-(N-pivaloyl)amino]phenylpropan-1,3-dione.

The obtained compound (0.4 g) was treated in a similar manner as in Example 64 to give 0.39 g of 2-(4-methoxy)phenyl-5-(N-pivaloyl)amino-4H-benzopyran-4-one.

The compound thus obtained was dissolved in 10 ml of dioxane and 5 ml of concentrated hydrochloric

acid was added to the solution, followed by stirring with heating under reflux for 5.5 hours. The reaction mixture was concentrated under reduced pressure, and the residue was purified by silica gel column chromatography (eluent; chloroform : methanol = 40 : 1) and then recrystallized from ethyl acetate-n-hexane to give 142 mg (yield 48%) of Compound 69.

Example 70

5-Amino-2-(4-bromo)phenyl-4H-benzopyran-4-one (Compound 70):

In 1 ml of toluene was suspended 115 mg of sodium hydride (60% oil dispersion), and a toluene solution of 500 mg of 2-ethoxycarbonyl-N-pivaloyl-3-(2-tetrahydropyranyl)oxyaniline and 285 mg of 4-bromoacetophenone was added to the suspension. The mixture was stirred at 100°C for 45 minutes, followed by addition of ethyl acetate. The reaction mixture was washed successively with water and a saturated aqueous solution of sodium chloride, dried over anhydrous sodium sulfate, and then concentrated under reduced pressure. The residue was purified by silica gel column chromatography (eluent; hexane : ethyl acetate = 4 : 1) to give 491 mg (yield 70%) of 1-(4-bromo)phenyl-3-[2-(2-tetrahydropyranyl)oxy-6-(N-pivaloyl)amino]phenyl propan-1,3-dione.

The obtained compound (233 mg) was dissolved in a solvent mixture of 10 ml of isopropanol and 10 ml of ethanol, and 250 mg of Amberlist 15 was added to the solution, followed by stirring with heating under reflux for 7 hours. After the reaction mixture was filtered, the filtrate was concentrated under reduced pressure and the residue was recrystallized from chloroform-ether-hexane to give 182 mg (yield 96%) of 2-(4-bromo)phenyl-5-(N-pivaloyl)amino-4H-benzopyran-4-one.

The compound thus obtained (100 mg) was treated in a similar manner as in Example 64 to give 52 mg (yield 57%) of the hydrochloride of Compound 70.

Table 3

| Example [Melting point (°C)] | Molecular Formula MS (m/z) | I R (KBr tablet) $cm^{-1}$ | N M R [Solvent] δ (ppm) |
|---|---|---|---|
| 1 [279 - 281] | $C_{25}H_{28}N_2O_4$ 420 ($M^+$) | 3342, 2968, 1688, 1640, 1603, 1505, 1385, 1317, 1301, 1155 | [$CDCl_3/CD_3OD$] 1.33(9H,s), 1.35(9H,s), 6.73(3H,s), 7.25 (1H,dd,J=1.0, 8.3Hz), 7.66(1H,t,J=8.4Hz), 7.77(2H,d,J=9.0Hz), 7.91(2H,d,J=9.0Hz), 8.68(1H,dd,J=1.0, 8.3Hz), 12.85(1H,s) |
| 2 (dihydro-chloride) [186 - 188] | $C_{15}H_{12}N_2O_2$ 252 ($M^+$) | 3310, 2724, 2550, 1624, 1578, 1486, 1185, 835 | [DMSO-$d_6$] 6.52(1H,dd,J=0.9, 8.3Hz), 6.59(1H,s), 6.64 (1H,dd,J=0.9, 8.1Hz), 6.91(2H,d,J=8.6Hz), 7.33(1H,t,J=8.2Hz), 7.83(2H,d,J=8.8Hz) |
| 3 [134 - 135] | $C_{19}H_{21}N_3O_2$ 323 ($M^+$) | 3392, 3288, 2850, 2822, 2778, 1630, 1588, 1552, 1470, 1402, 1348, 1263, 1188, 1040, 823 | [$CDCl_3/CD_3OD$] 2.33(6H,s), 2.64(2H,t,J=5.9Hz), 3.25(2H,t, J=5.9Hz), 6.42(1H,dd,J=0.7, 8.1Hz), 6.49 (1H,s), 6.67(3H,m), 7.29(1H,t,J=8.2Hz), 7.73(2H,d,J=9.0Hz) |
| 4 [189 - 190] | $C_{25}H_{28}N_2O_4$ 420 ($M^+$) | 3400, 2962, 1684, 1646, 1610, 1516, 1485, 1323, 1156 | [$CDCl_3$] 1.37(9H,s), 1.39(9H,s), 6.75(1H,s), 7.25 (1H,dd,J=1.0, 8.1Hz), 7.62(1H,m), 7.64 (1H,t,J=8.3Hz), 7.71(1H,td,J=1.0, 2.2, 6.6Hz), 8.19(1H,t,J=2.0Hz), 8.73(1H,dd, J=0.7, 8.3Hz), 12.81(1H,s) |

| Example [Melting point(°C)] | Molecular Formula MS(m/z) | I R (KBr tablet) $cm^{-1}$ | N M R [Solvent] δ (ppm) |
|---|---|---|---|
| 5 (dihydro-chloride) [238 - 239] | $C_{15}H_{12}N_2O_2$ 252($M^+$) | 3400, 2850, 1637, 1580, 1483, 1273, 801 | [DMSO-d6] 6.56(1H,dd,J=0.8, 8.3Hz), 6.64(1H,dd,J=0.8, 8.2Hz), 6.77(1H,s), 7.42(1H,t,J=9.4Hz), 7.44(1H,dd,J=1.3, 8.6Hz), 7.59(1H,t,J=8.0 Hz), 7.85(1H,s), 7.91(1H,d,J=8.1Hz) |
| 6 [ >300 ] | $C_{19}H_{16}N_2O_4$ 336($M^+$) | 3266, 3100, 1702, 1636, 1605, 1505, 1321, 1255, 841 | [DMSO-d6] 2.10(3H,s), 2.18(3H,s), 6.99(1H,s), 7.41 (1H,d,J=7.9Hz), 7.78(1H,t,J=7.5Hz), 7.89 (1H,d,J=8.1Hz), 8.08(2H,d,J=8.9Hz), 8.50 (1H,d,J=8.6Hz), 10.31(1H,s), 12.59(1H,s) |
| 7 [169 - 172] | $C_{18}H_{18}N_2O_2$ 294($M^+$) | 1634, 1611, 1590, 1513, 1436, 1374, 1335, 1251, 1198, 1169, 818 | [$CDCl_3$] 2.92(3H,d,J=5.1Hz), 3.06(6H,s), 6.35(1H,d, J=8.1Hz), 6.50(1H,s), 6.62(1H,dd,J=0.9, 8.2Hz), 6.74(2H,dd,J=2.1, 9.2Hz), 7.39(1H, t,J=8.2Hz), 7.77(2H,dd,J=2.1, 9.2Hz), 9.20 (1H,b) |
| 8 [229 - 230] | $C_{17}H_{16}N_2O_2$ 353($M^+$) | 3392, 3286, 1626, 1609, 1583, 1549, 1524, 1468, 1400, 1373, 1308, 1253, 1198, 813 | [$CDCl_3$] 3.06(6H,s), 6.41(1H,dd,J=0.9, 8.1Hz), 6.50 (1H,s), 6.68(1H,dd,J=1.1, 8.2Hz), 6.75(2H, dd,J=2.2, 9.2Hz), 7.29(1H,t,J=7.3Hz), 7.77 (2H,dd,J=2.2, 9.2Hz) |

| Example [Melting point(°C)] | Molecular Formula MS(m/z) | I R (KBr tablet) $cm^{-1}$ | N M R [Solvent] δ (ppm) |
|---|---|---|---|
| 9 [184 - 185] | $C_{16}H_{14}N_2O_2$ 339($M^+$) | 3370, 3302, 1646, 1591, 1548, 1528, 1471, 1398, 1251, 1187, 822 | [$CDCl_3$] 2.91(3H,d,J=2.0Hz), 4.28(1H,b), 6.41(1H,dd, J=1.0, 8.2Hz), 6.49(1H,s), 6.65(2H,d,J=8.8 Hz), 6.66(1H,dd,J=1.0, 8.2Hz), 8.29(1H,t, J=8.1Hz), 7.74(2H,d,J=8.8Hz) |
| 10 [165 - 166] | $C_{20}H_{20}N_2O_2$ 320($M^+$) | 3380, 3272, 1628, 1582, 1548, 1516, 1469, 1388, 1235, 1202, 1127, 834 | [$CDCl_3$] 1.67(6H,m), 3.35(4H,m), 6.41(1H,dd,J=0.9, 8.2Hz), 6.50(1H,s), 6.68(1H,dd,J=0.9, 8.3 Hz), 6.94(2H,d,J=9.5Hz), 7.28(1H,t,J=8.1 Hz), 7.76(2H,dd,J=2.1, 9.2Hz) |
| 11 [223 - 226 decomp.] | $C_{19}H_{19}N_3O_2$ 321($M^+$) | 3676, 1634, 1589, 1552, 1516, 1397, 1242, 900, 815 | [$CDCl_3/CD_3OD$] 3.05(4H,m), 3.33(4H,m), 6.48(1H,dd,J=0.7, 8.3Hz), 6.53(1H,s), 6.70(1H,dd,J=0.8, 8.2 Hz), 6.98(2H,d,J=9.2Hz), 7.33(1H,t,J=8.1 Hz), 7.82(2H,d,J=9.2Hz) |
| 12 [211 - 212] | $C_{19}H_{18}N_2O_3$ 322($M^+$) | 1693, 1594, 1511, 1503, 1368, 1303, 1289, 1259, 1198, 803 | [$CDCl_3$] 2.27(3H,s), 3.08(6H,s), 6.60(1H,s), 6.74 (2H,d,J=9.0Hz), 7.17(1H,dd,J=0.9, 8.3Hz), 7.58(1H,t,J=8.3Hz), 7.80(2H,d,J=9.0Hz), 8.60(1H,dd,J=1.0, 8.3Hz), 12.83(1H,s) |
| 13 [272 - 274] | $C_{17}H_{14}N_2O_3$ 294($M^+$) | 3432, 3258, 1689, 1633, 1591, 1556, 1532, 1472, 1415, 1329, 1265, 841 | [DMSO-$d_6$] 2.09(3H,s), 6.52(1H,dd,J=0.9, 8.3Hz), 6.65 (1H,dd,J=0.9, 8.1Hz), 6.71(1H,s), 7.35(1H, t,J=8.2Hz), 7.44(2H,b), 7.76(2H,d,J=8.8Hz), 7.98(2H,d,J=8.8Hz), 10.26(1H,s) |

| Example [Melting point(°C)] | Molecular Formula MS(m/z) | I R (KBr tablet) $cm^{-1}$ | N M R [Solvent] δ (ppm) |
|---|---|---|---|
| 14 [293 decomp.] | $C_{15}H_{12}N_2O_3$ 268($M^+$) | 3648, 3468, 3378, 1630, 1593, 1541, 1446, 1404, 1344, 1255, 1143, 802 | [DMSO-$d_6$] 5.77(2H,s), 6.18(1H,s), 6.21(1H,dd,J=2.2, 8.8Hz), 6.45(1H,dd,J=0.7, 7.7Hz), 6.56(1H, dd,J=0.9, 8.2Hz), 6.83(1H,d,J=2.4Hz), 7.26 (1H,t,J=8.2Hz), 7.36(2H,b), 7.58(1H,d,J= 8.8Hz), 10.18(1H,s) |
| 15 [194 - 195] | $C_{17}H_{16}N_2O_2$ 280($M^+$) | 3650, 3462, 3352, 1640, 1588, 1536, 1465, 1395, 1351, 1276, 1250, 1186, 820 | [$CDCl_3$] 1.29(3H,t,J=7.2Hz), 3.23(2H,m), 4.05(1H,b), 6.41(1H,dd,J=0.9, 8.2Hz), 6.48(1H,s), 6.56 (2H,b), 6.64(2H,d,J=7.0Hz), 6.67(1H,dd,J= 1.0, 8.3Hz), 7.29(1H,t,J=8.2Hz), 7.72(2H, d,J=7.0Hz) |
| 16 [177 - 178] | $C_{25}H_{28}N_2O_4$ 420($M^+$) | 3078, 2968, 2344, 1697, 1645, 1613, 1526, 1509, 1385, 1326, 1294, 1260, 1157, 1120, 801 | [$CDCl_3$] 1.10(6H,d,J=6.8Hz), 1.40(9H,s), 1.80(3H,s), 5.05(1H,m), 6.78(1H,s), 7.23(1H,dd,J=1.0, 8.6Hz), 7.30(2H,dd,J=2.0, 8.6Hz), 7.67(1H, t,J=8.4Hz), 8.00(2H,dd,J=2.0, 6.6Hz), 8.76 (1H,dd,J=1.0, 8.4Hz), 12.77(1H,s) |
| 17 [208 - 209] | $C_{20}H_{20}N_2O_3$ 336($M^+$) | 3408, 3298, 2974, 1642, 1592, 1556, 1469, 1416, 1321, 1180, 845 | [$CDCl_3$] 1.09(6H,d,J=6.8Hz), 1.79(3H,s), 5.05(1H,m), 6.47(1H,dd,J=0.9, 8.3Hz), 6.50(2H,b), 6.66 (1H,s), 6.71(1H,dd,J=1.0, 8.2Hz), 7.27(2H, dd,J=2.2, 8.6Hz), 7.35(1H,t,J=8.2Hz), 7.96(2H,dd,J=2.2, 8.6Hz) |

| Example [Melting point(°C)] | Molecular Formula MS(m/z) | I R (KBr tablet) $cm^{-1}$ | N M R [Solvent] δ (ppm) |
|---|---|---|---|
| 18 [172 - 173] | $C_{18}H_{18}N_2O_2$ 262($M^+$) | 3464, 3354, 2962, 1640, 1589, 1534, 1466, 1397, 1188, 825 | [$CDCl_3$] 1.16(6H,d,J=6.4Hz), 3.65(1H,m), 6.29(1H,d, J=7.7Hz), 6.48(1H,s), 6.48(1H,dd,J=0.9, 8.3Hz), 6.61(1H,dd,J=0.9, 8.3Hz), 6.66(2H, d,J=9.0Hz), 7.30(1H,t,J=8.2Hz), 7.39(2H,b), 7.74(2H,d,J=8.8Hz) |
| 19 [195 - 196] | $C_{19}H_{20}N_2O_4$ 340($M^+$) | 3188, 1648, 1590, 1520, 1470, 1395, 1352, 1198, 814 | [DMSO-d6] 3.55(8H,m), 4.78(2H,b), 6.53(1H,s), 6.59 (1H,dd,J=0.7, 8.3Hz), 6.62(1H,dd,J=0.8, 8.2Hz), 6.82(2H,d,J=9.0Hz), 7.30(1H,t,J= 8.2Hz), 7.34(2H,b), 7.79(2H,d,J=9.0Hz) |
| 20 [220 - 221] | $C_{24}H_{26}Cl_2N_2O_3$ 461($M^+$+1) 463($M^+$+1) | 3066, 2956, 1678, 1641, 1605, 1518, 1384, 1253, 1182, 822 | [$CDCl_3$] 1.39(9H,s), 3.69(4H,t,J=7.1Hz), 3.84(4H,t, J=7.0Hz), 6.62(1H,s), 6.80(2H,d,J=9.0Hz), 7.18(1H,dd,J=1.0, 8.3Hz), 7.61(1H,t,J=8.4 Hz), 7.84(2H,d,J=9.0Hz), 8.71(1H,dd,J=1.0, 8.3Hz), 12.95(1H,s) |
| 21 [176 - 177] | $C_{19}H_{18}Cl_2N_2O_2$ 376($M^+$) 378($M^+$) 380($M^+$) | 3472, 3308, 2346, 1636, 1604, 1552, 1519, 1469, 1395, 1186, 819 | [DMSO-d6] 3.80(8H,m), 6.50(1H,dd,J=0.9, 8.2Hz), 6.58 (1H,s), 6.63(1H,dd,J=0.9, 8.1Hz), 6.90(2H, d,J=9.2Hz), 7.32(1H,t,J=8.2Hz), 7.41(2H,b), 7.85(2H,d,J=9.0Hz) |

| Example [Melting point(°C)] | Molecular Formula MS(m/z) | I R (KBr tablet) $cm^{-1}$ | N M R [Solvent] δ (ppm) |
|---|---|---|---|
| 22 (1.2 hydro-chloride) [159 - 162] | $C_{18}H_{18}N_2O_2$ 294($M^+$) | 1641, 1599, 1484, 1390, 1189, 1098, 801 | [DMSO-$d_6$] 0.95(3H,t,J=7.3Hz), 2.50(2H,m), 3.07(2H,t, J=7.1Hz), 6.50(1H,s), 6.51(1H,d,J=8.2Hz), 6.64(1H,d,J=8.2Hz), 6.72(2H,d,J=8.8Hz), 7.31(1H,t,J=8.2Hz), 7.76(2H,d,J=8.9Hz) |
| 23 [138 - 139] | $C_{19}H_{20}N_2O_2$ 308($M^+$) | 3454, 3354, 2952, 2930, 2862, 2830, 1639, 1589, 1530, 1462, 1391, 1353, 1247, 1183, 824 | [$CDCl_3$] 0.97(3H,t,J=7.2Hz), 1.45(2H,m), 1.63(2H, m), 3.18(2H,q,J=6.4Hz), 4.10(1H,b), 6.41 (1H,dd,J=0.9, 8.3Hz), 6.48(1H,s), 6.51(2H, b), 6.64(2H,d,J=9.0Hz), 6.67(1H,dd,J=0.9, 8.2Hz), 7.28(1H,t,J=8.2Hz), 7.22(2H,d,J= 8.8Hz) |
| 24 (1.9 hydro-chloride) [157 - 159] | $C_{22}H_{18}N_2O_2$ 342($M^+$) | 2836, 2514, 2408, 1638, 1608, 1532, 1480, 1391, 1187, 834 | [DMSO-$d_6$] 4.37(2H,s), 6.48(1H,s), 6.50(1H,dd,J=0.9, 8.3Hz), 6.61(1H,dd,J=0.9, 8.1Hz), 6.71(2H, d,J=9.0Hz), 7.30(1H,t,J=8.4Hz), 7.36(5H,s), 7.73(2H,d,J=9.0Hz) |
| 25 [205 - 210] | $C_{17}H_{14}N_2O_4$ 310($M^+$) | 3388, 2346, 1724, 1635, 1586, 1532, 1469, 1404, 1234, 1191, 826 | [DMSO-$d_6$] 3.84(2H,b), 6.49(1H,dd,J=0.7, 8.0Hz), 6.51 (1H,s), 6.62(1H,dd,J=0.7, 8.1Hz), 7.68(2H, d,J=9.0Hz), 7.30(1H,t,J=8.2Hz), 7.39(2H,b), 7.76(2H,d,J=8.8Hz) |

| Example [Melting point(°C)] | Molecular Formula MS(m/z) | I R (KBr tablet) $cm^{-1}$ | N M R [Solvent] δ (ppm) |
|---|---|---|---|
| 26 [151 - 153] | $C_{19}H_{18}N_2O_4$ 338($M^+$) | 3366, 2962, 1726, 1634, 1589, 1533, 1470, 1392, 1257, 1214, 1187, 825 | [$CDCl_3$] 1.32(3H,t,J=7.1Hz), 3.97(2H,d,J=5.1Hz), 4.28(2H,q,J=7.2Hz), 4.73(1H,b), 6.41(1H,d, J=8.2Hz), 6.50(1H,s), 6.66(2H,d,J=8.8Hz), 6.67(1H,dd,J=1.0, 8.2Hz), 7.29(1H,t,J=8.3 Hz), 7.75(2H,d,J=8.8Hz) |
| 27 [207 - 209] | $C_{18}H_{16}N_2O_4$ 324($M^+$) | 3378, 2942, 1732, 1641, 1591, 1532, 1395, 1185, 825 | [$CDCl_3$] 3.82(3H,s), 3.99(2H,d,J=5.3Hz), 4.72(1H,b), 6.42(1H,dd,J=0.9, 8.3Hz), 6.50(1H,s), 6.67 (2H,d,J=8.8Hz), 6.67(1H,dd,J=1.0, 8.0Hz), 7.30(1H,t,J=8.3Hz), 7.75(2H,d,J=9.0Hz) |
| 28 [193 - 194] | $C_{17}H_{16}N_2O_3$ 296($M^+$) | 3402, 3296, 2934, 1635, 1612, 1581, 1552, 1476, 1402, 1329, 1305, 1246, 1188, 825 | [DMSO-$d_6$] 3.19(2H,q,J=5.9Hz), 3.58(2H,q,J=5.5Hz), 4.71(1H,t,J=5.4Hz), 6.42(1H,t,J=5.6Hz), 6.48(1H,dd,J=1.0, 8.2Hz), 6.49(1H,s), 6.61 (1H,dd,J=0.9, 8.1Hz), 6.60(2H,d,J=9.0Hz), 7.30(1H,t,J=8.3Hz), 7.39(2H,b), 7.74(2H,d, J=8.8Hz) |
| 29 [260 decomp.] | $C_{15}H_{12}N_2O_3$ 268($M^+$) | 3392, 3348, 3288, 1635, 1590, 1539, 1471, 1451, 1418, 1298, 1245, 839 | [DMSO-$d_6$] 5.34(2H,b), 6.35(1H,s), 6.41(1H,dd,J=0.9, 8.3Hz), 6.69(2H,d,J=8.2Hz), 7.23(1H,d,J= 2.0Hz), 7.29(1H,dd,J=2.0, 8.3Hz), 7.30(1H, t,J=8.1Hz), 7.34(2H,b), 9.42(1H,s) |

| Example [Melting point(°C)] | Molecular Formula MS(m/z) | I R (KBr tablet) $cm^{-1}$ | N M R [Solvent] δ (ppm) |
|---|---|---|---|
| 30 [206 - 207] | $C_{25}H_{27}FN_2O_4$ 438($M^+$) | 3676, 2966, 1690, 1645, 1610, 1513, 1385, 1257, 1156, 839 | [$CDCl_3$] 1.37(9H,s), 1.39(9H,s), 6.68(1H,s), 7.21 (1H,dd,J=1.0, 8.3Hz), 7.64(1H,t,J=8.4Hz), 7.67(1H,d,J=12.7Hz), 7.71(1H,dd,J=2.0, 8.6 Hz), 7.81(1H,d,J=3.9Hz), 8.61(1H,t,J=8.3 Hz), 8.73(1H,dd,J=1.0, 8.6Hz), 12.79(1H,s) |
| 31 (1.3 hydro-chloride) [215 - 217] | $C_{15}H_{11}FN_2O_2$ 270($M^+$) | 3436, 3318, 2850, 1640, 1625, 1524, 1485, 1395, 1322, 1254, 835 | [DMSO-$d_6$] 6.52(1H,dd,J=0.9, 8.3Hz), 6.58(1H,s), 6.66 (1H,dd,J=0.9, 8.1Hz), 7.32(1H,t,J=8.2Hz), 7.60(1H,dd,J=2.0, 8.6Hz), 7.69(1H,dd,J=2.0, 12.8Hz), 7.88(1H,t,J=8.9Hz) |
| 32 [235 - 237] | $C_{23}H_{26}F_2N_2O_3$ 416($M^+$) | 3526, 3220, 2964, 1680, 1645, 1609, 1514, 1439, 1385, 1351, 1303, 1258, 1041, 854, 764 | [$CDCl_3$] 1.38(9H,s), 1.39(9H,s), 6.65(1H,s), 7.15 (1H,s), 7.19(1H,dd,J=1.0, 8.3Hz), 7.47(2H, dd,J=3.2, 8.6Hz), 7.51(1H,t,J=8.4Hz), 8.71 (1H,dd,J=0.9, 8.4Hz), 12.67(1H,b) |
| 33 (0.9 hydro-chloride) [222 - 225] | $C_{15}H_{10}F_2N_2O_2$ 260($M^+$) | 3470, 3310, 2834, 2576, 1621, 1528, 1486, 1454, 1392, 1359, 1282, 1148, 960, 808 | [DMSO-$d_6$] 6.52(1H,dd,J=0.7, 8.3Hz), 6.69(1H,s), 6.69 (1H,dd,J=0.9, 8.1Hz), 7.33(1H,t,J=8.2Hz), 7.65(2H,dd,J=2.7, 10.4Hz) |

| Example [Melting point(°C)] | Molecular Formula MS(m/z) | I R (KBr tablet) $cm^{-1}$ | N M R [Solvent] δ (ppm) |
|---|---|---|---|
| 34 [147 - 148] | $C_{18}H_{18}N_2O_3$ 310($M^+$) | 3340, 1642, 1591, 1470, 1394, 1251, 1185, 1084, 819 | [$CDCl_3$] 3.37(2H,q,J=5.3Hz), 3.41(3H,s), 3.63(2H,t, J=5.1Hz), 4.86(1H,b), 6.41(1H,dd,J=0.9, 8.2Hz), 6.48(1H,s), 6.51(2H,b), 6.67(1H, dd,J=0.9, 8.2Hz), 6.67(2H,dd,J=2.8, 9.0Hz), 7.29(1H,t,J=8.2Hz), 7.72(2H,dd,J=2.0, 8.8Hz) |
| 35 [286 - 287] | $C_{26}H_{30}N_2O_5$ 450($M^+$) | 3226, 2952, 1676, 1643, 1596, 1513, 1417, 1243, 1187, 1169, 1077, 1060, 833 | [$CDCl_3$] 1.35(9H,s), 1.38(9H,s), 4.01(3H,s), 6.71 (1H,s), 7.20(1H,d,J=9.0Hz), 7.51(1H,s), 7.72(2H,d,J=8.8Hz), 7.94(2H,d,J=8.8Hz), 8.68(1H,d,J=9.0Hz), 12.52(1H,b) |
| 36 [216 - 217] | $C_{16}H_{14}N_2O_3$ 282($M^+$) | 3420, 3334, 1641, 1600, 1562, 1515, 1446, 1395, 1247, 1189, 1065, 831 | [DMSO-$d_6$] 3.84(3H,s), 5.92(2H,b), 6.43(1H,d,J=8.8Hz), 6.50(1H,s), 6.68(2H,d,J=8.8Hz), 6.89(2H,b), 7.18(1H,d,J=8.8Hz), 7.71(2H,d,J=8.8Hz) |
| 37 (1.7 hydrochloride) [203 - 205] | $C_{17}H_{16}N_2O_3$ 296($M^+$) | 3320, 2540, 1640, 1606, 1569, 1527, 1503, 1444, 1386, 1283, 1257, 1193, 830 | [DMSO-$d_6$] 2.78(3H,s), 3.94(3H,s), 6.70(2H,d,J=9.2Hz), 6.72(1H,s), 6.92(1H,d,J=8.8Hz), 7.35(1H,d, J=9.0Hz), 7.83(2H,d,J=9.0Hz) |
| 38 [138 - 139] | $C_{17}H_{15}BrN_2O_2$ 358($M^++1$) 360($M^++1$) | 3384, 3284, 1634, 1590, 1530, 1462, 1398, 1253, 1190, 820 | [$CDCl_3$] 3.40-3.78(4H,m), 4.48(1H,b), 6.40(1H,d,J= 9.0Hz), 6.47(1H,s), 6.66(1H,d,J=7.0Hz), 6.68(2H,d,J=9.0Hz), 7.29(1H,t,J=8.1Hz), 7.73(2H,d,J=8.8Hz) |

| Example [Melting point(°C)] | Molecular Formula MS(m/z) | I R (KBr tablet) $cm^{-1}$ | N M R [Solvent] δ (ppm) |
|---|---|---|---|
| 39 [162 - 163] | $C_{17}H_{15}ClN_2O_2$ 314($M^+$+1) 316($M^+$+1) | 3390, 1645, 1592, 1532, 1399, 1275, 1253, 1188, 820 | [DMSO-$d_6$] 3.51(2H,q,J=6.0Hz), 3.74(2H,t,J=6.1Hz), 5.77(1H,dd,J=0.9, 8.3Hz), 6.49(1H,s), 6.62 (1H,dd,J=0.8, 8.1Hz), 6.68(1H,t,J=5.8Hz), 6.74(2H,d,J=8.8Hz), 7.30(1H,t,J=8.2Hz), 7.40(2H,b), 7.77(2H,d,J=8.8Hz) |
| 40 [150 - 152] | $C_{17}H_{14}N_2O_2$ 278($M^+$) | 3408, 3302, 2984, 1628, 1584, 1554, 1510, 1470, 1423, 1339, 1296, 1248, 908, 837 | [$CDCl_3$] 2.19(4H,s), 6.43(1H,dd,J=0.8, 8.2Hz), 6.52 (2H,b), 6.54(1H,s), 6.68(1H,dd,J=0.9, 8.2 Hz), 7.11(2H,dd,J=1.8, 8.6Hz), 7.31(1H,t, J=8.2Hz), 7.77(2H,dd,J=2.0, 8.8Hz) |
| 41 [291 - 293] | $C_{22}H_{22}N_2O_4$ 378($M^+$) | 3280, 3102, 2970, 1704, 1679, 1641, 1605, 1511, 1321, 1300, 1254, 1189, 1162, 916, 841 | [DMSO-$d_6$] 1.29(9H,s), 2.10(3H,s), 6.96(1H,s), 7.39 (1H,dd,J=1.0, 8.3Hz), 7.45(1H,t,J=8.3Hz), 7.79(2H,d,J=9.3Hz), 8.06(2H,dd,J=1.7, 8.8 Hz), 8.57(1H,dd,J=0.7, 8.3Hz), 10.28(1H,b), 12.87(1H,b) |
| 42 [150 - 153] | $C_{29}H_{30}N_2O_6S$ 534($M^+$) | 3350, 2964, 1642, 1597, 1513, 1385, 1305, 1247, 1188, 978, 824 | [$CDCl_3$] 1.38(9H,s), 2.41(3H,s), 3.50(2H,q,J=5.5Hz), 4.23(2H,t,J=5.2Hz), 4.51(1H,b), 6.57(1H,s), 6.59(2H,d,J=8.6Hz), 7.14(1H,d,J=8.6Hz), 7.30(2H,d,J=7.5Hz), 7.59(1H,t,J=8.6Hz), 7.69(2H,d,J=6.2Hz), 7.78(2H,d,J=5.7Hz), 8.69(1H,d,J=8.4Hz), 12.94(1H,b) |

| Example [Melting point(°C)] | Molecular Formula MS(m/z) | I R (KBr tablet) $cm^{-1}$ | N M R [Solvent] δ (ppm) |
|---|---|---|---|
| 43 [123 - 124] | $C_{31}H_{28}N_2O_3$ 476($M^+$) | 3478, 3308, 1640, 1602, 1581, 1520, 1470, 1394, 1351, 1307, 1209, 1122, 825 | [$CDCl_3$] 3.75(4H,s), 4.53(2H,s), 4.71(2H,s), 6.40 (1H,dd,J=0.9, 8.3Hz), 6.47(1H,s), 6.49(2H, b), 6.65(1H,dd,J=0.9, 8.5Hz), 6.77(2H,dd, J=2.0, 9.2Hz), 7.17-7.37(11H,m), 7.71(2H, dd,J=1.9, 9.0Hz) |
| 44 [145 - 146] | $C_{24}H_{22}N_2O_3$ 386($M^+$) | 3460, 3348, 2866, 1640, 1590, 1537, 1398, 1347, 1250, 1189, 1114, 823 | [$CDCl_3$] 3.40(2H,q,J=5.3Hz), 3.73(2H,t,J=5.3Hz), 4.50(1H,b), 4.57(2H,s), 6.41(1H,dd,J=0.9, 8.1Hz), 6.49(1H,s), 6.50(2H,b), 6.66(2H, dd,J=1.9, 8.8Hz), 6.67(1H,dd,J=0.9, 8.3Hz), 7.33(1H,t,J=8.1Hz), 7.35(5H,s), 7.72(2H, dd,J=2.0, 9.0Hz) |
| 45 [143 - 144] | $C_{19}H_{20}N_2O_3$ 324($M^+$) | 3462, 3354, 2850, 1640, 1590, 1535, 1458, 1397, 1345, 1249, 1187, 1110, 824 | [$CDCl_3$] 1.24(3H,t,J=7.0Hz), 3.37(2H,q,J=5.3Hz), 3.55(2H,q,J=7.0Hz), 3.63(2H,t,J=5.2Hz), 4.51(1H,t,J=4.5Hz), 6.41(1H,dd,J=0.9, 8.2 Hz), 6.49(1H,s), 6.50(2H,b), 6.65(1H,m), 6.67(2H,dd,J=1.6, 8.6Hz), 7.29(1H,t,J=8.1 Hz), 7.72(2H,dd,J=1.9, 8.8Hz) |
| 46 [147 - 148] | $C_{22}H_{22}N_2O_5$ 394($M^+$) | 2978, 2934, 1698, 1668, 1650, 1612, 1531, 1506, 1384, 1298, 1084, 842 | [$CDCl_3$] 1.59(3H,s), 2.29(3H,s), 3.32(3H,s), 3.56 (2H,t,J=5.4Hz), 3.92(2H,t,J=5.5Hz), 6.76 (1H,s), 7.23(1H,dd,J=1.0, 8.3Hz), 7.44(2H, dd,J=2.0, 8.5Hz), 7.67(1H,t,J=8.4Hz), 7.98 (2H,dd,J=2.0, 8.6Hz), 8.67(1H,dd,J=0.9, 8.3Hz), 12.58(1H,b) |

| Example [Melting point(°C)] | Molecular Formula MS(m/z) | I R (KBr tablet) $cm^{-1}$ | N M R [Solvent] δ (ppm) |
|---|---|---|---|
| 47 [250 decomp.] | $C_{15}H_{12}N_2O_3$ 268($M^+$) | 3320, 2540, 1640, 1606, 1569, 1503, 1444, 1386, 1283, 1257, 1193, 1064, 830 | [DMSO-d6] 5.88(2H,s), 6.35(1H,d,J=8.6Hz), 6.45(1H,s), 6.66(2H,d,J=8.8Hz), 6.71(2H,s), 6.99(1H,d, J=8.8Hz), 7.78(2H,d,J=8.8Hz), 8.72(1H,b) |

| Example [Melting point(°C)] | Elemental Analysis (%) [upper column: calcd. lower column: found] | I R (KBr tablet) $cm^{-1}$ | N M R [Solvent] δ (ppm) |
|---|---|---|---|
| 48 [258 - 260] | — | 1666, 1638, 1601, 1510, 1458, 1426, 1385, 1332, 1300 | [DMSO-$d_6$] 1.30(9H,s), 6.82(1H,s), 6.95(2H,d,J=9.0Hz), 7.31(1H,dd,J=1.1, 8.3Hz), 7.69 (1H,t,J=8.3Hz), 7.92(2H,d,J=8.8Hz), 8.57(1H,dd,J=1.1, 8.3Hz) |
| 49 [244 - 245] | $C_{15}H_{11}NO_3 \cdot HCl \cdot 0.8H_2O$<br>C H N<br>59.24 4.51 4.61<br>59.27 4.76 4.67 | 1634, 1607, 1579, 1515, 1482, 1442, 1386 | [DMSO-$d_6$] 6.59(1H, dd,J=1.1, 8.1Hz), 6.62(1H,s), 6.71(1H,dd,J=0.9, 8.1Hz), 6.95(2H,d, J=8.9Hz), 7.46(1H,dd,J=8.1, 8.3Hz), 7.86(2H,d,J=8.8Hz) |
| 50 [256 - 257] | $C_{15}H_{11}NO_3 \cdot HCl$<br>C H N<br>62.19 4.17 4.83<br>62.24 4.12 4.96 | 1625, 1573, 1485, 1446, 1394, 1344, 1280 | [DMSO-$d_6$] 6,61(1H,dd,J=0.9, 8.1Hz), 6.65(1H,s), 6.69(1H,d,J=8.1Hz), 6.93-7.06(1H,m), 7.29-7.46(4H,m) |
| 51 [280 - 281] | $C_{15}H_{11}NO_4 \cdot HCl \cdot 0.1H_2O$<br>C H N<br>58.58 4.00 4.55<br>58.55 4.05 4.27 | 1630, 1605, 1574, 1485, 1453, 1395, 1334, 1300 | [DMSO-$d_6$] 6.47(1H,d,J=2.2Hz), 6.50(1H,s), 6.60 (1H,dd,J=1.0, 8.0Hz), 6.76(1H,dd,J=1.0, 8.2Hz), 6.82(2H,d,J=2.2Hz), 7.37 (1H,dd,J=8.1, 8.1Hz) |
| 52 [256 - 258] | $C_{19}H_{20}N_2O_3 \cdot 2HCl \cdot 0.9H_2O$<br>C H N<br>55.19 5.80 6.77<br>55.34 6.19 6.81 | 1637, 1590, 1540, 1510, 1474, 1426, 1396, 1330, 1310 | [DMSO-$d_6$] 2.26(6H,s), 2.68(2H,t,J=5.9Hz), 4.15 (2H,t,J=5.8Hz), 6.52(1H,dd,J=1.1, 8.1 Hz), 6.63(1H,dd,J=1.1, 8.1Hz), 6.66 (1H,s), 7.08(2H,d,J=9.0Hz), 7.33(1H, dd,J=8.1, 8.3Hz), 7.95(2H,d,J=8.8Hz) |

| Example [Melting point(°C)] | Elemental Analysis (%) [upper column: calcd. lower column: found] | I R (KBr tablet) $cm^{-1}$ | N M R [Solvent] δ (ppm) |
|---|---|---|---|
| 53 [210 - 212] | $C_{19}H_{20}N_2O_3 \cdot 2HCl \cdot 0.3H_2O$<br>C H N<br>56.67 5.66 6.96<br>56.76 6.09 6.73 | 1636, 1587, 1553, 1486, 1387, 1319 | [DMSO-$d_6$] 2.39(6H,s), 2.81(2H,t,J=5.7Hz), 4.16 (2H,t,J=5.7Hz), 6.44(1H,dd,J=0.9, 8.1 Hz), 6.60(1H,s), 6.68(1H,dd,J=1.0, 8.1 Hz), 6.99-7.11(1H,m), 7.32(1H,t,J=8.3 Hz), 7.42-7.56(3H,m) |
| 54 | — | — | [$CDCl_3$] 1.38(9H,s), 3.96(3H,s), 3.98(6H,s), 6.67(1H,s), 6.96(1H,d,J=8.5Hz), 7.18 (1H,d,J=9.1Hz), 7.44(1H,d,J=2.0Hz), 7.59(1H,dd,J=2.1, 8.4Hz), 8.66(1H,d, J=9.2Hz) |
| 55 [241 - 244] | $C_{18}H_{17}NO_5 \cdot HCl \cdot 0.4H_2O$<br>C H N<br>58.27 5.11 3.78<br>58.31 5.08 3.69 | 1637, 1602, 1578, 1556, 1515, 1438, 1385, 1357 | [$CDCl_3$] 3.87(3H,s), 3.89(3H,s), 3.96(3H,s), 7.01(1H,s), 7.05(1H,d,J=8.5Hz), 7.14 (1H,d,J=8.4Hz), 7.39(1H,d,J=8.7Hz), 7.56(1H,d,J=1.5Hz), 7.67(1H,dd,J=1.7, 8.4Hz) |
| 56 [224 - 225] | $C_{16}H_{13}NO_3 \cdot HCl$<br>C H N<br>63.27 4.64 4.61<br>63.36 4.66 4.53 | 1645, 1558, 1505, 1451, 1389, 1357 | [DMSO-$d_6$] 3.96(3H,s), 6.98(1H,s), 7.00(1H,d,J= 8.6Hz), 7.39(1H,d,J=8.7Hz), 7.56-7.67 (3H,m), 8.00-8.11(2H,m) |

| Example [Melting point(°C)] | Elemental Analysis (%) [upper column: calcd. lower column: found] | I R (KBr tablet) $cm^{-1}$ | N M R [Solvent] δ (ppm) |
|---|---|---|---|
| 57 [230 decomp.] | $C_{17}H_{15}NO_4\cdot HCl\cdot 0.1H_2O$<br>C H N<br>60.84 4.87 4.17<br>60.81 4.86 4.12 | 1637, 1608, 1578, 1553, 1504, 1463, 1429, 1382, 1357 | [$CDCl_3$] 3.89(3H,s), 3.92(3H,s), 6.39(1H,d,J=8.8Hz), 6.64(1H,s), 6.98-7.11(1H,m), 7.08(1H,d,J=8.8Hz), 7.32-7.58(3H,m) |
| 58 [228 decomp.] | $C_{17}H_{15}NO_4\cdot HCl\cdot 0.3H_2O$<br>C H N<br>60.20 4.93 4.13<br>60.25 4.81 4.07 | 1645, 1603, 1576, 1538, 1506, 1386 | [DMSO-$d_6$] 3.87(3H,s), 3.97(3H,s), 6.90(1H,s), 7.07(1H,d,J=8.8Hz), 7.11(2H,d,J=9.0Hz), 7.39(1H,d,J=9.0Hz), 8.00(2H,d,J=9.0Hz) |
| 59 [188 - 190] | $C_{16}H_{13}NO_4\cdot 0.4HCl\cdot 0.5H_2O$<br>C H N<br>66.06 4.98 4.90<br>66.13 4.68 4.90 | 1645, 1605, 1585, 1487, 1387, 1318 | [$CDCl_3$] 3.87(3H,s), 6.44(1H,dd,J=0.9, 8.1Hz), 6.60(1H,s), 6.68(1H,dd,J=0.9, 8.1Hz), 6.97-7.10(1H,m), 7.32(1H,t,J=8.3Hz), 7.25-7.44(2H,m) |
| 60 [191 - 193] | $C_{15}H_{11}NO_2\cdot HCl\cdot 0.2H_2O$<br>C H N<br>64.97 4.51 5.05<br>64.86 4.45 5.20 | 1644, 1487, 1452, 1393 | [$CDCl_3$] 6.24(1H,dd,J=0.9, 8.1Hz), 6.62(1H,s), 6.69(1H,dd,J=0.9, 8.1Hz), 7.24(1H,d, J=8.1Hz), 7.42-7.53(3H,m), 7.83-7.93 (2H,m) |
| 61 [160 - 161] | $C_{15}H_{12}N_2O_2\cdot HCl$<br>C H N<br>62.40 4.54 9.70<br>63.62 4.52 9.60 | 1635, 1595, 1581, 1508, 1496, 1450, 1414, 1387, 1334 | [DMSO-$d_6$] 6.67(1H,d,J=7.9Hz), 6.80(1H,s), 7.01 (1H,d,J=8.4Hz), 7.48-7.59(4H,m), 7.96 -8.07(2H,m) |

| Example [Melting point(°C)] | Elemental Analysis (%) [upper column: calcd. lower column: found] | I R (KBr tablet) $cm^{-1}$ | N M R [Solvent] δ (ppm) |
|---|---|---|---|
| 62 [228 - 230] | $C_{19}H_{20}N_2O_2 \cdot HCl \cdot 0.4H_2O$<br>C H N<br>64.82 6.24 7.96<br>64.76 6.05 7.89 | 1647, 1605, 1593, 1521, 1495, 1451, 1430, 1334 | [$CDCl_3$] 2.33(6H,s), 2.63(2H,t,J=6.6Hz), 3.28 (2H,t,J=6.4Hz), 6.39(1H,d,J=8.4Hz), 6.60(1H,s), 6.62(1H,dd,J=0.9, 7.9Hz), 7.30-7.56(4H,m), 7.82-7.93(2H,m) |
| 63 [241 - 242] | — | 1667, 1645, 1607, 1522, 1492, 1450, 1418, 1382 | [DMSO-$d_6$] 1.96(3H,s), 6.68(1H,d,J=8.1Hz), 6.91 (1H,s), 6.96(1H,d,J=8.1Hz), 7.46-7.64 (4H,m), 8.01-8.09(2H,m) |
| 64 [161 - 163] | $C_{17}H_{15}NO_4 \cdot HCl \cdot 0.4H_2O$<br>C H N<br>59.88 4.97 4.11<br>59.92 4.98 3.93 | 1627, 1600, 1513, 1485, 1424, 1384, 1335, 1267, 1156 | [DMSO-$d_6$] 3.85(3H,s), 3.88(3H,s), 6.52(1H,dd,J=0.9,8.2Hz), 6.69(1H,dd,J=1.0, 8.2Hz), 6.81(1H,s), 7.11(1H,d,J=8.6Hz), 7.32 (1H,t,J=8.2Hz), 7.53(1H,d,J=2.0Hz), 7.64(1H,dd,J=2.2, 8.6Hz) |
| 65 [203 - 204] | $C_{17}H_{15}NO_5 \cdot 0.9HCl$<br>C H N<br>58.99 4.63 4.05<br>58.92 4.54 3.85 | 1625, 1516, 1484, 1430, 1395, 1344, 1293, 1269, 1121 | [DMSO-$d_6$] 3.88(6H,s), 6.54(1H,dd,J=0.7, 8.2Hz), 6.74(1H,dd,J=0.9, 8.0Hz), 6.83(1H,s), 7.28(2H,s), 7.35(1H,t,J=8.2Hz) |
| 66 [155 - 156] | $C_{17}H_{15}NO_4 \cdot 1.5HCl$<br>C H N<br>58.01 4.72 3.98<br>58.31 4.40 3.86 | 1634, 1605, 1557, 1470, 1374, 1294, 1253, 1219, 1131 | [DMSO-$d_6$] 3.87(3H,s), 3.93(3H,s), 6.51(1H,dd,J=0.8, 8.3Hz), 6.61(1H,dd,J=0.8, 8.2Hz), 6.71(1H,d,J=1.3Hz), 6.73(1H,dd,J=1.5, 9.9Hz), 6.75(1H,s), 7.32(1H,t,J=8.3Hz), 7.85(1H,d,J=9.0Hz) |

| Example [Melting point(°C)] | Elemental Analysis (%) [upper column: calcd. lower column: found] | I R (KBr tablet) $cm^{-1}$ | N M R [Solvent] δ (ppm) |
|---|---|---|---|
| 67 [195 - 196] | $C_{17}H_{15}NO_4 \cdot 0.9HCl \cdot H_2O$<br>C H N<br>58.65 5.18 4.02<br>58.60 5.07 3.87 | 3340, 2532, 1631, 1457, 1432, 1388, 1326, 1205, 1167 | [DMSO-$d_6$] 3.84(6H,s), 6.55(1H,dd,J=0.9, 8.2Hz), 6.72(1H,dd,J=0.9, 8.2Hz), 6.88(1H,s), 7.15(2H,d,J=2.4Hz), 7.36(1H,t,J=8.2Hz) |
| 68 [200 decomp.] | $C_{15}H_{11}NO_4$<br>C H N<br>66.91 4.12 5.20<br>66.22 4.14 4.50 | 3374, 1642, 1594, 1544, 1444, 1405, 1339, 1256 | [DMSO-$d_6$] 6.42(1H,dd,J=2.2, 8.8Hz), 6.48(1H,d, J=2.2Hz), 6.49(1H,dd,J=0.7, 8.1Hz), 6.59(1H,dd,J=0.7, 8.1Hz), 6.87(1H,s), 7.30(1H,t,J=8.2Hz), 7.72(1H,d,J=8.5Hz) |
| 69 [143 - 145] | $C_{16}H_{13}NO_3$<br>C H N<br>71.90 4.90 5.24<br>71.98 4.88 5.12 | 3384, 1647, 1604, 1512, 1473, 1396, 1325, 1263, 1185 | [$CDCl_3$] 3.88(3H,s), 6.43(1H,dd,J=0.9, 8.1Hz), 6.55(1H,s), 6.69(1H,dd,J=0.9, 8.3Hz), 7.00(2H,dd,J=2.2, 6.9Hz), 7.31(1H,t, J=8.2Hz), 7.84(2H,dd,J=2.2, 6.9Hz) |
| 70 [197 - 199] | $C_{15}H_{10}BrNO_2 \cdot 0.5HCl$<br>C H N<br>53.88 3.16 4.19<br>54.07 2.85 4.00 | 3392, 2570, 1638, 1587, 1553, 1484, 1412, 1386 | [DMSO-$d_6$] 6.55(1H,dd,J=0.9, 8.2Hz), 6.68(1H,dd, J=1.0, 8.4Hz), 6.85(1H,s), 7.37(1H,t, J=8.3Hz), 7.77(2H,d,J=8.6Hz), 7.99(2H, d,J=9.0Hz) |

Reference Example 1

1-Acetyl-4-(2-tetrahydropyranyl)oxybenzene (Compound a):

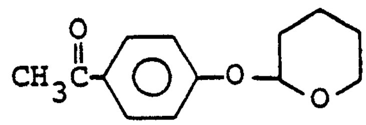

4-Acetylphenol (10.0 g), 10.1 ml of 2,3-dihydropyrane and 1.5 g of pyridinium p-toluenesulfonate were heated in 150 ml of methylene chloride with stirring for 4.5 hours. The reaction mixture was cooled and washed once with water, a 5% aqueous solution of sodium bicarbonate and water, respectively. The methylene chloride layer was decolored with activated charcoal and then dried over anhydrous sodium sulfate. After filtration, the solvent was evaporated and the residue was crystallized from hexane to give 13.6 g (yield 84%) of Compound a.
NMR ($CDCl_3$)δ(ppm): 1.65-1.94(6H, m), 2.55(3H, s), 3.61-3.90(3H, m), 5.51(1H, brs), 7.07(2H, d, J=9.0Hz), 7.91(2H, d, J=9.0Hz)

Reference Example 2

1-(3-Methoxy)phenyl-3-[2-(2-tetrahydropyranyl)oxy-6-(N-pivaloyl)amino]phenylpropan-1,3-dione (Compound b):

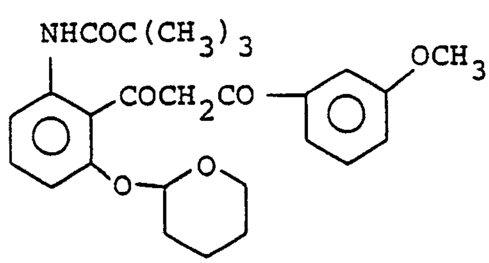

In 3 ml of dioxane was suspended 0.34 g of sodium hydride (60% oil dispersion). A solution of 1.50 g of 2-ethoxycarbonyl-N-pivaloyl-3-(2-tetrahydropyranyl)oxyaniline and 0.64 g of 3-methoxyacetophenone in 4 ml of dioxane was added dropwise to the suspension with heating under reflux. The mixture was heated under reflux for further 30 minutes. Water was added to the reaction mixture, followed by washing with petroleum ether. To the aqueous layer was added ethyl acetate to carry out extraction. The ethyl acetate layer was washed with a saturated aqueous solution of sodium chloride, dried over anhydrous sodium sulfate, and then concentrated under reduced pressure. The residue was purified by silica gel column chromatography (eluent; hexane : ethyl acetate = 3 : 1) to give 0.95 g (yield 49%) of Compound b.
NMR ($CDCl_3$)δ(ppm): 1.29(9H, s), 1.5-1.83(6H, m), 3.57-3.86(2H, m), 3.86(3H, s), 5.51(1H, b), 6.86(1H, s), 6.94(1H, dd), 7.03(1H, dd), 7.26-7.53(4H, m), 8.09(1H, dd), 10.09(1H, s), 16.10 (1H, b)

Reference Example 3

3,5-Dimethoxy-4-(2-tetrahydropyranyl)oxyacetophenone (Compound c)

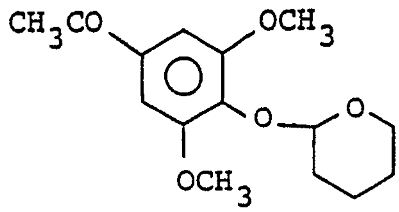

3,5-Dimethoxy-4-hydroxyacetophenone (5.0 g) was dissolved in 50 ml of methylene chloride, and 4.65 ml of 2,3-dihydropyrane and 0.52 g of pyridinium p-toluenesulfonate were added to the solution. The mixture was stirred with heating under reflux for 16.5 hours. The reaction mixture was washed successively with a 5% aqueous solution of sodium bicarbonate, and water, dried over anhydrous sodium sulfate, and then concentrated under reduced pressure. The residue was purified by silica gel column chromatography (eluent; hexane : ethyl acetate = 2 : 1) to give 1.75 g (yield 25%) of Compound c.
NMR ($CDCl_3$)δ(ppm): 1.64-2.04(6H, m), 2.57(3H, s), 3.48-3.79(2H, m), 3.90(6H, s), 5.58(1H, b), 7.22 (2H, s)

Reference Example 4

2,4-Dimethoxymethyloxyacetophenone (Compound d):

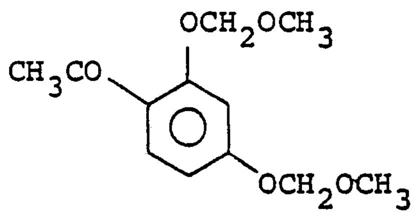

2,4-Dihydroxyacetophenone (3.0 g) was dissolved in 50 ml of tetrahydrofuran, and 3.3 ml of chloromethyl methyl ether and 1.73 g of sodium hydride (60% oil dispersion) were added to the solution under ice cooling. The reaction mixture was stirred for one hour under ice cooling and then poured into ice water, followed by extraction with chloroform. The chloroform layer was dried over anhydrous sodium sulfate and concentrated under reduced pressure. The residue was purified by silica gel column chromatography (eluent; hexane : ethyl acetate = 4 :1 to 3 :1) to give 3.80 g (yield 80%) of Compound d.
NMR ($CDCl_3$)δ(ppm): 2.60(3H, s), 3.48(3H, s), 3.52 (3H, s), 5.19(2H, s), 5.26(2H, s), 6.65-6.83 (2H, m), 7.77-(1H, d)

## Claims

1. A compound of formula (I):

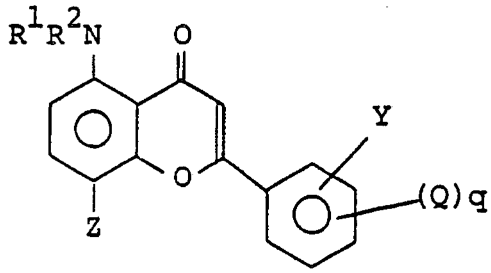

(I)

wherein

$R^1$ and $R^2$ independently represent a member selected from the group consisting of hydrogen, lower alkyl, cycloalkyl, lower alkanoyl, amino, lower alkanoylamino, benzyl and $-(CH_2)_nX$ wherein X represents a member selected from the group consisting of hydroxyl, lower alkanoyloxy, lower alkoxyl, cycloalkyloxy, benzyloxy, mercapto, lower alkylthio, halogen, carboxyl, lower alkoxycarbonyl, lower alkylsulfonyloxy, trifluoromethanesulfonyloxy, optionally substituted arylsulfonyloxy, $-CONR^3R^4$ (wherein $R^3$ and $R^4$ independently represent hydrogen or lower alkyl) and $-NR^3R^4$ (wherein $R^3$ and $R^4$ have the same significances as defined above); and n is an integer of 1 to 6;
Y represents a member selected from the group consisting of hydrogen, $-NR^{1a}R^{2a}$ (wherein $R^{1a}$ and $R^{2a}$ have the same significances as $R^1$ and $R^2$ described above),

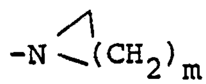

(wherein m represents an integer of 1 to 5),

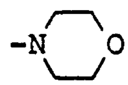 and 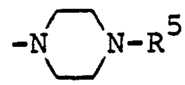

(wherein $R^5$ represents hydrogen, lower alkyl, lower alkanoyl or benzyl);
Q represents a member selected from the group consisting of lower alkyl, hydroxyl, lower alkoxyl, lower alkanoyloxy, halogen and $-O(CH_2)_{\ell}X^1$ (wherein $X^1$ has the same significance as X described above and $\ell$ represents an integer of 1 to 6) and Qs are the same or different from each other;
q represents an integer of 0 to 4; and
Z represents a member selected from the group consisting of hydrogen, hydroxyl, lower alkoxyl and lower alkanoyloxy
or a pharmaceutically acceptable salt thereof.

2. A compound according to claim 1, wherein the alkyl moiety in said lower alkyl, lower alkoxyl, lower alkylthio, lower alkoxycarbonyl and lower alkylsulfonyloxy is a straight-chain or branched alkyl group having 1 to 6 carbon atoms.

3. A compound according to claim 1, wherein the cycloalkyl moiety in said cycloalkyl and cycloalkyloxy is a cycloalkyl group having 3 to 6 carbon atoms.

4. A compound according to claim 1, wherein the alkanoyl moiety in said lower alkanoyl, lower alkanoyloxy and lower alkanoylamino is a straight-chain or branched alkanoyl group having 1 to 6 carbon atoms.

5. A compound according to claim 1, wherein the aryl moiety in said optionally substituted arylsulfonyloxy is selected from the group consisting of phenyl and tolyl.

6. A compound according to claim 1, wherein said halogen is selected from the group consisting of fluorine, chlorine, bromine and iodine.

7. A compound according to claim 1, wherein both $R^1$ and $R^2$ are hydrogen and Y is $-NR^{1a}R^{2a}$.

8. A compound according to claim 7, wherein both $R^{1a}$ and $R^{2a}$ are hydrogen.

9. A compound according to claim 8, wherein Q is fluorine, q is 0, 1 or 2 and Z is hydrogen or methoxy.

10. A compound according to claim 7, wherein one of $R^{1a}$ and $R^{2a}$ is hydrogen and the other is selected from the group consisting of methyl, ethyl, isopropyl, methoxycarbonylmethyl, β-chloroethyl and ethoxyethyl.

11. A compound according to claim 10, wherein q is 0 and Z is hydrogen.

12. A compound according to claim 1, wherein both $R^1$ and $R^2$ are hydrogen, Y is

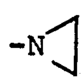 ;

q is 0 and Z is hydrogen.

European Patent Office

# EUROPEAN SEARCH REPORT

Application number

EP 89123374.4

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
| --- | --- | --- | --- |
| A | EP - A2/3 - 0 237 986 (SHIONOGI & CO) * Claims 1,6 * -- | 1 | C 07 D 311/30<br>C 07 D 405/10<br>//A 61 K 31/35 |
| A | DE - A1 - 3 601 417 (A. NATTERMANN & CIE) * Abstract * ---- | 1 | |

TECHNICAL FIELDS SEARCHED (Int Cl 5)

C 07 D 311/00
C 07 D 405/00

The present search report has been drawn up for all claims

| Place of search | Date of completion of the search | Examiner |
| --- | --- | --- |
| VIENNA | 09-03-1990 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document